# EUROPEAN PATENT APPLICATION

(11) **EP 4 722 234 A1**
(43) Date of publication of application: **08.04.2026**
(21) Application number: 24819559.6
(22) Date of filing: 04.06.2024
(51) Int. Cl.: C07K 14/47, C07K 7/06, C12N 15/86, A61P 9/00, A61K 38/00

(54) **GENE CONSTRUCT COMPRISING NUCLEOTIDES ENCODING DECOY PEPTIDES FOR INHIBITING PROTEIN PHOSPHATASE 1-MEDIATED DEPHOSPHORYLATION OF PHOSPHOLAMBAN AND NUCLEOTIDES ENCODING CCN5 PROTEIN OR FRAGMENTS THEREOF, AND USE THEREOF**

(30) Priority: 05.06.2023 KR 20230072147
(71) Applicant: BethphaGen Inc., Gwangju 61005 (KR)
(72) Inventor: JANG, Seung Pil, Gwangju 61019 (KR); KWAK, Taehwan, Yongin-si, Gyeonggi-do 17103 (KR)
(74) Representative: Cabinet Becker et Associés
(86) International application number: PCT/KR2024/007650
(87) International publication number: WO 2024/253408

(57) **Abstract**

The present invention relates to: a gene construct comprising nucleotides encoding decoy peptides for inhibiting protein phosphatase 1 (PP1)-mediated dephosphorylation of phospholamban (PLB) and nucleotides encoding the CCN5 protein or a fragment thereof; and a pharmaceutical composition for preventing and treating heart diseases, the pharmaceutical composition comprising the same. The pharmaceutical composition for preventing and treating heart diseases according to the present invention simultaneously expresses a decoy peptide inhibiting PP1-mediated dephosphorylation and the CCN5 protein, thereby exhibiting a synergistic therapeutic effect, and thus can be effectively used for preventing or treating heart diseases.

## Description

### Technical Field

The present invention relates to a pharmaceutical composition for preventing and treating a heart disease. Specifically, the present invention relates to: a gene construct comprising a nucleotide encoding a decoy peptide that inhibits protein phosphatase 1 (PP1)-mediated dephosphorylation of phospholamban (PLB); and a nucleotide encoding a CCN5 protein or a fragment thereof; and a pharmaceutical composition for preventing or treating a heart disease, comprising the same as an active ingredient.

### Background Art

Heart failure (HF) is a disease characterized by structural and functional impairment of the pumping function of the ventricle, which fills or ejects blood, resulting in complex symptoms. Heart failure has a mortality rate of over 50% within five years of diagnosis, making it a disease with a higher mortality rate than cancer. The number of heart failure patients worldwide is estimated to be approximately 38 million, and its prevalence is increasing with the aging population. However, treatment for heart failure has no fundamental treatment method and can only slow the progression of the disease. Therefore, medical management incurs significant costs, placing a significant burden on patients (Braunwald E., Lancet, 385(9970):812-824, 2015).

The causes of heart failure include heart conditions, genetic defects, and systemic diseases. Representative diseases related to the causes of heart failure include ischemic, hypertensive, and valvular heart diseases. In addition, they include primary cardiomyopathies of genetic or acquired origin, secondary cardiomyopathies including amyloidosis, congenital heart disease, and pericardial disease (Maron BJ. et al., Circulation, 113(14):1807-1816, 2006). Heart failure resulting from various cardiac diseases results in pathological cardiac remodeling, which manifests as structural alterations and functional abnormalities in the heart. Specifically, cellular remodeling occurs due to alterations in the size, shape, and function of cardiomyocytes, while tissue remodeling occurs due to excessive accumulation of extracellular matrix (ECM), leading to fibrosis of cardiac tissue. This pathological cardiac remodeling involves pathological changes in the transcriptional, signaling, structural, electrophysiological, and functional roles of cardiomyocytes.

The extracellular matrix of the cardiac muscle is a sophisticated structure that supports the mechanical functions of cardiomyocytes, enabling efficient contraction and relaxation. It also facilitates appropriate force transmission, electrical signal transmission, intercellular communication, and metabolite exchange within the cardiac muscle microenvironment. Increased stress, injury, and disease of the cardiac wall lead to fibrosis in the extracellular matrix, which impairs the motor functions such as contraction and relaxation of cardiomyocytes and myofibrils (Li AH. et al., Circ Res., 114(5):916-927, 2014).

In addition, pathological remodeling leads to dysfunction of cardiac muscle contraction and relaxation, as well as cardiomyocyte death. At the cellular level, cardiomyocyte hypertrophy and death affect myocardial excitation-contraction coupling (EC) and lead to pathological changes in the molecular mechanisms that regulate cardiomyocyte contraction, cell survival, mitochondrial function related to energy metabolism, and oxidative stress.

Pathological structural changes in cardiac tissue occur, including the generation and fibrosis of myofibroblasts, stiffening of vascular smooth muscle, dysfunction of vascular endothelial cells, and the promotion of inflammatory responses of immune cells. This cellular disease progression leads to tissue-level remodeling through an integrated process of cardiomyocyte hypertrophy and death, vascular loss, fibrosis, inflammation, metabolic dysfunction, and electrophysiological remodeling, ultimately leading to heart failure.

Neurohormonal blockers have been used for the treatment of heart failure for over 40 years. However, despite the efficacy of heart failure therapeutic agents in alleviating symptoms and reducing cardiac overload stress, the prognosis for heart failure patients remains extremely pessimistic, with mortality rates reaching 50% at 5 years after onset and 90% at 10 years after onset. In addition, once heart failure progresses to severe stages, the only treatment options are cardiac assist devices and heart transplantation. Therefore, the development of novel therapies that fundamentally treat heart failure and restore the heart is urgently needed.

Among the newly understood disease mechanisms in the field of heart failure therapeutic agent development, it has been revealed that cardiac remodeling, including fibrosis, which directly impacts cardiac pump function, and cardiomyocyte damage interact to form a vicious cycle that closely influences the onset, progression, and prognosis of the disease.

Therefore, treating a single drug target or disease mechanism may result in insufficient results in the treatment of heart disease. Therefore, research is needed on combination therapeutic agents that address both functional restoration at the cellular level and cardiac histological remodeling.

### Detailed Description of Invention

### Technical Problem

Accordingly, the present inventors have conducted research to develop an effective therapeutic agent for heart disease. As a result, the present inventors have found that a gene construct, a recombinant vector, and a recombinant virus, which comprise a nucleotide encoding a decoy peptide that inhibits protein phosphatase 1 (PP1)-mediated dephosphorylation of phospholamban (PLB) and a nucleotide encoding a CCN5 protein or a fragment thereof, exhibited synergistic therapeutic effects on heart disease in mouse models of heart failure caused by various etiologies. Based on the above, the present inventors have completed the present invention.

### Solution to Problem

In one aspect of the present invention, there is provided a gene construct comprising a nucleotide encoding a decoy peptide that inhibits protein phosphatase 1 (PP1)-mediated dephosphorylation of phospholamban (PLB); and a nucleotide encoding a CCN5 protein or a fragment thereof.

In another aspect of the present invention, there is provided a recombinant vector comprising the gene construct.

In another aspect of the present invention, there is provided a recombinant virus comprising the gene construct.

In another aspect of the present invention, there is provided a pharmaceutical composition comprising the gene construct, the recombinant vector, or the recombinant virus as an active ingredient.

In another aspect of the present invention, there is provided a method for preventing or treating a heart disease, comprising administering the pharmaceutical composition to a subject.

In another aspect of the present invention, there is provided a method for preventing or treating a heart disease, comprising administering to a subject a recombinant vector comprising a nucleotide encoding a decoy peptide that inhibits protein phosphatase 1 (PP1)-mediated dephosphorylation of phospholamban (PLB), or a recombinant virus comprising the same; and administering to a subject step a recombinant vector comprising a nucleotide encoding a CCN5 protein or a fragment thereof, or a recombinant virus comprising the same.

In another aspect of the present invention, there is provided a use of the pharmaceutical composition for the prevention or treatment of a heart disease.

In another aspect of the present invention, there is provided a use of the pharmaceutical composition for the manufacture of a medicament for preventing or treating a heart disease.

### Effects of Invention

The present invention relates to a gene construct comprising a nucleotide encoding a decoy peptide that inhibits protein phosphatase 1 (PP1)-mediated dephosphorylation of phospholamban (PLB); and a nucleotide encoding a CCN5 protein or a fragment thereof; and a pharmaceutical composition comprising the same. The pharmaceutical composition according to the present invention simultaneously expresses a decoy peptide that inhibits PP1-mediated dephosphorylation and a CCN5 protein, thereby exhibiting a synergistic therapeutic effect, and thus has a remarkable effect in preventing or treating a heart disease.

### Brief Description of Drawings

Figure 1a is a schematic diagram showing the structure of the pUC-scCMV-SPTATm3-SE-P2A-CCN5 vector.
Figure 1b is a schematic diagram showing the structure of the pUC-scCMV-SPTATm3-SESE-P2A-CCN5 vector.
Figure 1c is a schematic diagram showing the structure of the pUC-scCMV-SPTATm3-SESESE-P2A-CCN5 vector.
Figure 1d is a schematic diagram showing the structure of the pUC-scCMV-SPTATm3-SESESE-dNP2-SESESE-CCN5 vector.

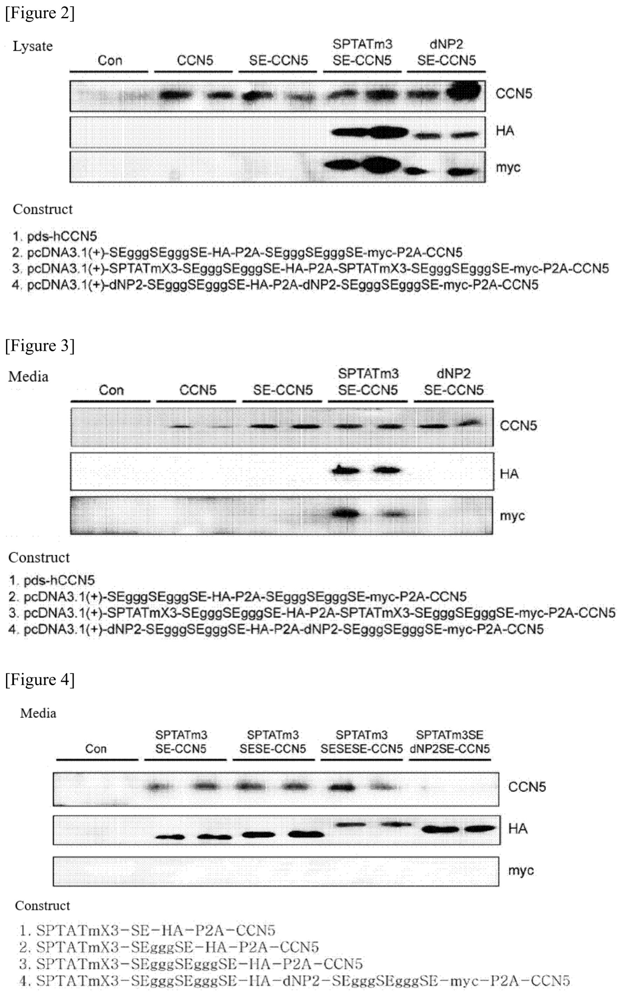

Figure 2 shows the results obtained by confirming the decoy peptide and the CCN5 protein in a lysate to confirm whether the decoy peptide and the CCN5 protein are expressed in cells by the vector of the present invention. CCN5 represents a CCN5-only construct; SE-CCN5 represents a CCN5 multiple expression construct using an SE peptide sequence (decoy peptide sequence) without a CPP and with P2A; SPTATm3-SE-CCN5 represents a CCN5 multiple expression construct using an SE peptide, SPTATm3 as a CPP, and P2A; and dNP2-SE-CCN5 represents a CCN5 multiple expression construct using an SE peptide, dNP2 as a CPP, and P2A.
Figure 3 shows the results obtained by confirming the decoy peptide and the CCN5 protein in a medium to confirm whether the decoy peptide and the CCN5 protein of the present invention is secreted to the outside of cells. CCN5 represents a CCN5-only construct; SE-CCN5 represents a CCN5 multiple expression construct using an SE peptide sequence (decoy peptide sequence) without a CPP and with P2A; SPTATm3-SE-CCN5 represents a CCN5 multiple expression construct using an SE peptide, SPTATm3 as a CPP, and P2A; and dNP2-SE-CCN5 represents a CCN5 multiple expression construct using an SE peptide, dNP2 as a CPP, and P2A.
Figure 4 shows the results obtained by confirming whether the expression level of CCN5 protein increases depending on the repeats of the nucleotides encoding the decoy peptide. SPTATm3-SE-CCN5 represents a CCN5 multiple expression construct using an SE peptide sequence (decoy peptide sequence) and P2A; SPTATm3-SESE-CCN5 represents a CCN5 multiple expression construct using a peptide sequence with two repeats of the SE peptide sequence and P2A; SPTATm3-SESESE-CCN5 represents a CCN5 multiple expression construct using a peptide sequence with three repeats of the SE peptide sequence and P2A; and SPTATm3-dNP2SE-CCN5 represents a CCN5 multiple expression construct using a peptide sequence with three repeats of the SE peptide sequence using SPTATm3 and dNP2 as a CPP and P2A.

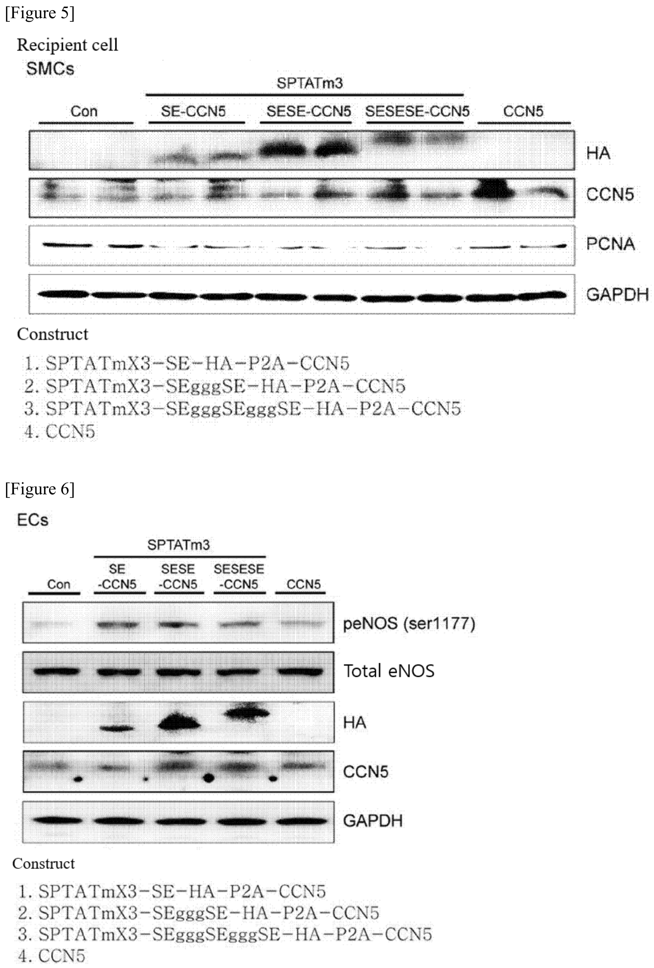

Figure 5 shows the results obtained by confirming the degree to which the decoy peptide and the CCN5 protein are introduced into smooth muscle cells (SMCs) depending on the repeats of the nucleotides encoding the decoy peptide (p value: **<0.005, *<0.05, n.s>0.05). SE-CCN5 represents a CCN5 multiple expression construct using an SE peptide sequence and P2A; SESE-CCN5 represents a CCN5 multiple expression construct using a peptide sequence with two repeats of the SE sequence and P2A; SESESE-CCN5 represents a CCN5 multiple expression construct using a peptide sequence with three repeats of the SE sequence and P2A; and CCN5 represents a CCN5-only construct.
Figure 6 shows the results obtained by confirming the degree to which the decoy peptide and the CCN5 protein are introduced into endothelial cells (ECs) depending on the repeats of the nucleotides encoding the decoy peptide (p value: **<0.005, *<0.05, n.s>0.05). SE-CCN5 represents a CCN5 multiple expression construct using an SE peptide sequence and P2A; SESE-CCN5 represents a CCN5 multiple expression construct using a peptide sequence with two repeats of the SE sequence and P2A; SESESE-CCN5 represents a CCN5 multiple expression construct using a peptide sequence with three repeats of the SE sequence and P2A; and CCN5 represents a CCN5-only construct.

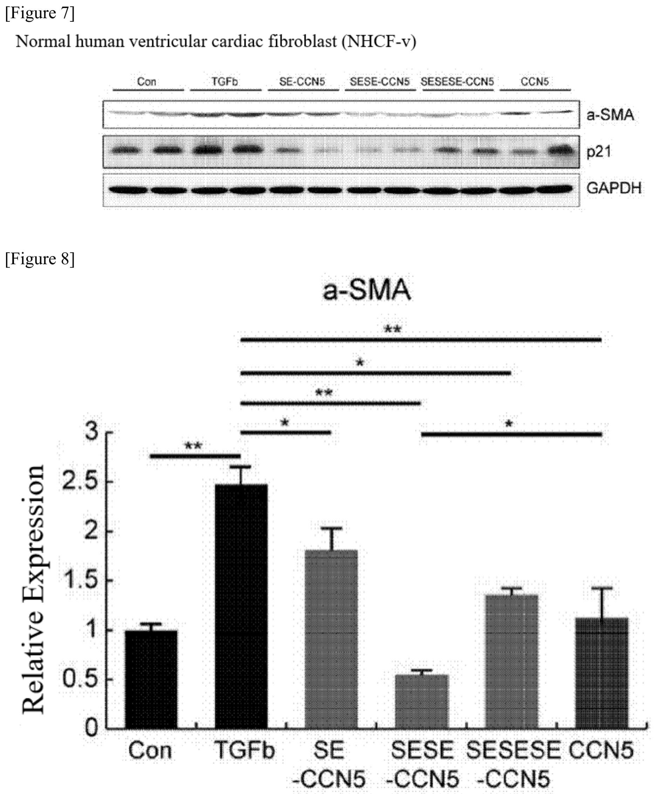

Figure 7 shows the results obtained by confirming the protein expression levels of a-SMA, p21, and GAPDH depending on the repeats of the nucleotides encoding the decoy peptide after treatment of normal human ventricular cardiac fibroblasts (NHCF-v) with TGF beta, followed by treatment with the decoy peptide and the CCN5 protein (p value: **<0.005, *<0.05, n.s>0.05). SE-CCN5 represents a CCN5 multiple expression construct using an SE peptide sequence and P2A; SESE-CCN5 represents a CCN5 multiple expression construct using a peptide sequence with two repeats of the SE sequence and P2A; SESESE-CCN5 represents a CCN5 multiple expression construct using a peptide sequence with three repeats of the SE sequence and P2A; and CCN5 represents a CCN5-only construct.
Figure 8 is a graph showing the mRNA expression of a-SMA, as determined by qRT-PCR, in an experimental group in which NHCF-v was treated with a medium containing the decoy peptide and the CCN5 protein (p value: **<0.005, *<0.05, n.s>0.05). SE-CCN5 represents a CCN5 multiple expression construct using an SE peptide sequence and P2A; SESE-CCN5 represents a CCN5 multiple expression construct using a peptide sequence with two repeats of the SE sequence and P2A; SESESE-CCN5 represents a CCN5 multiple expression construct using a peptide sequence with three repeats of the SE sequence and P2A; and CCN5 represents a CCN5-only construct.

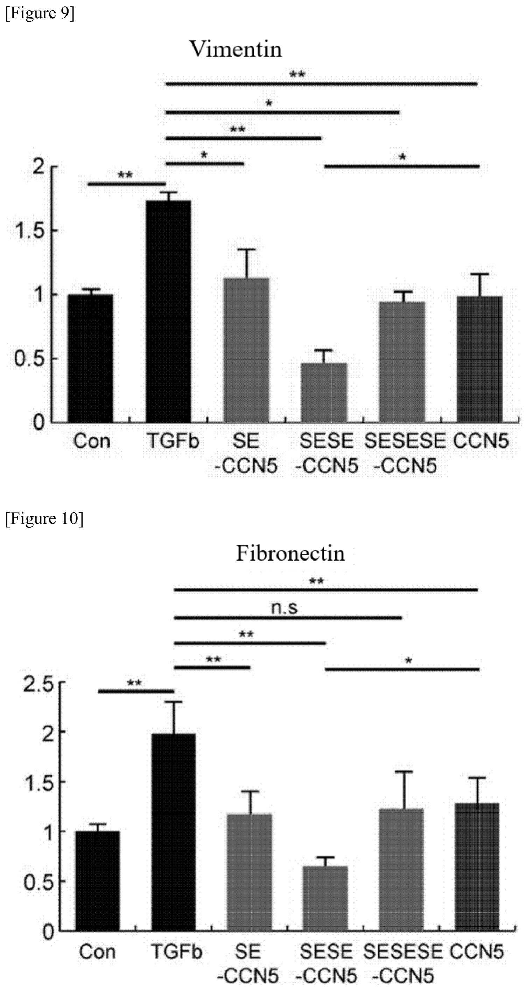

Figure 9 is a graph showing the mRNA expression of vimentin, as determined by qRT-PCR, in an experimental group in which NHCF-v was treated with a medium containing the decoy peptide and the CCN5 protein (p value: **<0.005, *<0.05, n.s>0.05). SE-CCN5 represents a CCN5 multiple expression construct using an SE peptide sequence and P2A; SESE-CCN5 represents a CCN5 multiple expression construct using a peptide sequence with two repeats of the SE sequence and P2A; SESESE-CCN5 represents a CCN5 multiple expression construct using a peptide sequence with three repeats of the SE sequence and P2A; and CCN5 represents a CCN5-only construct.
Figure 10 is a graph showing the mRNA expression of fibronectin, as determined by qRT-PCR, in an experimental group in which NHCF-v was treated with a medium containing the decoy peptide and the CCN5 protein (p value: **<0.005, *<0.05, n.s>0.05). SE-CCN5 represents a CCN5 multiple expression construct using an SE peptide sequence and P2A; SESE-CCN5 represents a CCN5 multiple expression construct using a peptide sequence with two repeats of the SE sequence and P2A; SESESE-CCN5 represents a CCN5 multiple expression construct using a peptide sequence with three repeats of the SE sequence and P2A; and CCN5 represents a CCN5-only construct.
Figure 11a is a schematic diagram showing the structure of the construct BTG011, which simultaneously expresses the SPTATm3-SESE peptide and the CCN5 protein via the CMV promoter.

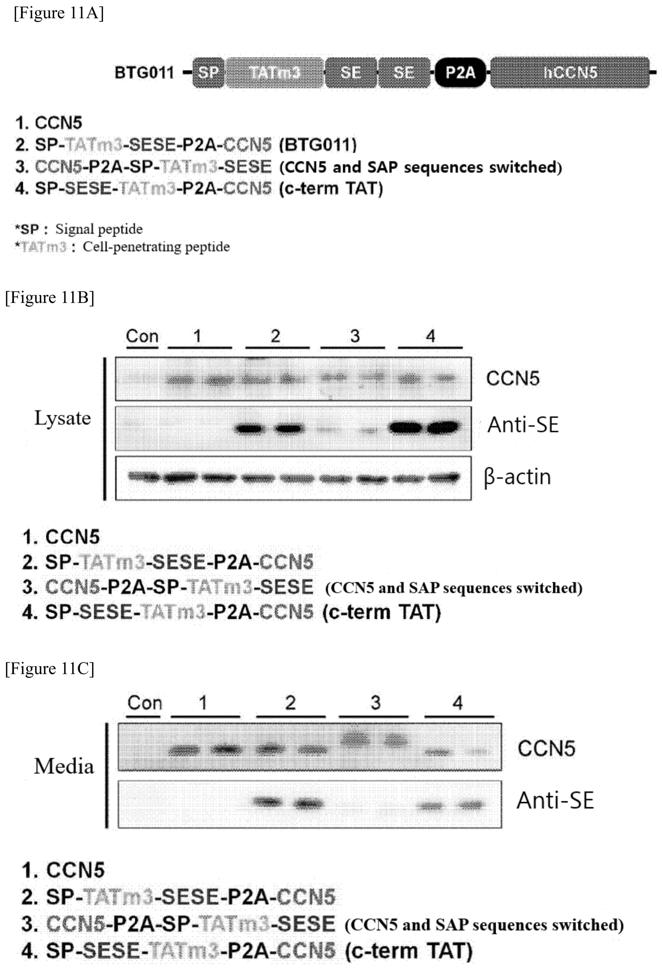

Figure 11b shows the results obtained by confirming the decoy peptide and the CCN5 protein in a lysate to confirm whether the decoy peptide and the CCN5 protein are expressed in cells by the vector of the present invention. CCN5 represents a CCN5-only construct; SPTATm3-SESE-P2A-CCN5 represents a CCN5 multiple expression construct using an SE peptide sequence comprising an N-terminal CPP and P2A; CCN5-P2A-SP-TATm3-SESE represents an SE peptide multiple expression construct comprising a CPP using CCN5 and P2A; and SP-SESE-TATm3-P2A-CCN5 represents a CCN5 multiple expression construct using an SE peptide sequence comprising a C-terminal CPP and P2A.
Figure 11c shows the results obtained by confirming the decoy peptide and the CCN5 protein in a medium to confirm whether the decoy peptide and the CCN5 protein of the present invention is secreted to the outside of cells. CCN5 represents a CCN5-only construct; SPTATm3-SESE-P2A-CCN5 represents a CCN5 multiple expression construct using an SE peptide sequence comprising an N-terminal CPP and P2A; CCN5-P2A-SP-TATm3-SESE represents an SE peptide multiple expression construct comprising a CPP using CCN5 and P2A; and SP-SESE-TATm3-P2A-CCN5 represents a CCN5 multiple expression construct using an SE peptide sequencecomprising a C-terminal CPP and P2A.
Figure 12 is a graph showing myocardial contractility after injection of BTG001 (expressing CCN5 only), BTG010 (expressing CPP and decoy peptide (TATm3-SESE)), and BTG011 (expressing TATm3-SESE and CCN5) AAV viruses at different doses in an AngII-induced heart failure mouse model (p value: **<0.005, n = 3-6).
Figure 13 is a histological image showing the degree of cardiac fibrosis after injection of BTG001, BTG010, and BTG011 AAV viruses at different doses in an AngII-induced heart failure mouse model.
Figure 14 is a graph showing the degree of cardiac fibrosis, as quantified by ImageScope, after injection of BTG001, BTG010, and BTG011 AAV viruses at different doses in an AngII-induced heart failure mouse model (p value: **<0.005, n = 3-6).
Figure 15 is a graph showing the heart weight/body weight ratio in mice after injection of BTG001, BTG010, and BTG011 AAV viruses in a transverse aortic constriction (TAC)-induced heart failure model (p value: **<0.005, *<0.05, n = 4-7).
Figure 16 is a graph showing the cardiac contractility (fractional shortening) in mice after injection of BTG001, BTG010, and BTG011 AAV viruses in a TAC-induced heart failure model (p value: **<0.005, *<0.05, n = 4-7).
Figure 17 is a histological image showing the degree of cardiac fibrosis after injection of BTG001, BTG010, and BTG011 AAV viruses at different doses in a TAC-induced heart failure model.
Figure 18 is a graph showing the degree of cardiac fibrosis quantified after injection of BTG001, BTG010, and BTG011 AAV viruses in a TAC-induced heart failure model (p value: *<0.05, n = 3-4).
Figure 19 is a schematic diagram of TAT and TATm3.

### Best Mode for Carrying out the Invention

### Novel gene construct

In one aspect of the present invention, there is provided a gene construct comprising a nucleotide encoding a decoy peptide that inhibits protein phosphatase 1 (PP1)-mediated dephosphorylation of phospholamban (PLB); and a nucleotide encoding a CCN5 protein or a fragment thereof.

### Decoy peptide

As used herein, the term "decoy peptide" refers to a peptide having the activity of inhibiting protein phosphatase 1 (PP1)-mediated dephosphorylation of phospholamban (PLB). The decoy peptide may be referred to as an SE peptide. Specifically, the decoy peptide of the present invention, designed to mimic phosphorylated PLB, inhibits PP1-mediated dephosphorylation of PLB, significantly increases the phosphorylation level of PLB, and increases contractile parameters, thereby significantly improving left ventricular diastolic pressure.

The decoy peptide may consist of an amino acid sequence represented by the following Structural formula (I):

X1-Ala-X2-X3-Ile-Glu-X4 (I)

wherein X1 is 0 to 50 amino acid residues,
X2 is Ser, Glu, or Asp,
X3 is Thr or Glu,
X4 is 0 to 50 amino acid residues, and
when X3 is Thr, X2 is not Ser.

At this time, the decoy peptide can inhibit protein phosphatase 1 (PP1)-mediated dephosphorylation of phospholamban (PLB) by competitive inhibition.

In the decoy peptide of the present invention, the "Ala-X2-X3-Ile-Glu" sequence is essential for the action and function of the decoy peptide. The X1 and X4 residues can be variously modified. In this regard, the present invention may also encompass any peptide or polypeptide comprising the "Ala-X2-X3-Ile-Glu" sequence, as long as it retains function or activity as a decoy peptide against PP1.

As used herein, the term "peptide" refers to a linear molecule formed by amino acid residues linked together by peptide bonds.

As used herein, the term "polypeptide" refers to a polymer of identical or different amino acids linked by peptide bonds.

As used herein, the term "decoy peptide" refers to a peptide or polypeptide designed to comprise a peptide sequence that mimics the short loop of phosphorylated PLB, which can competitively bind to PP1 and thereby block its action.

As used herein, the term "PP1-mediated dephosphorylation" refers to the dephosphorylation of PLB by PP1. At this time, PLB is of human origin, and the NCBI Accession Number for its amino acid sequence is AAA60109.1, AAA60083.1, or AAD55950.1.

As used herein, the term "competitive inhibition" refers to inhibition of dephosphorylation by competitive binding to PP1 for the formation of a decoy peptide-PP1 complex. The decoy peptide can bind to PP1 by competing with the phosphorylation site on PLB for binding to PP1.

The decoy peptide includes any decoy peptide of any length that inhibits PP1-mediated dephosphorylation of PLB. For example, the decoy peptide of the present invention may be 5 to 100 amino acids, 5 to 80 amino acids, 5 to 60 amino acids, 5 to 40 amino acids, 5 to 30 amino acids, 5 to 20 amino acids, 5 to 15 amino acids, 5 to 9 amino acids, or 6 to 9 amino acids in length.

The decoy peptide is interpreted as including a functional equivalent of the decoy peptide of the present invention.

As used herein, the term "functional equivalent" refers to a modification of the amino acid sequence of the decoy peptide that has similar or improved biological activity compared to the decoy peptide of the present invention, while also including amino acid substitutions, additions, or deletions in the amino acid sequence of the decoy peptide of the present invention (e.g., modifications of amino acid residues surrounding the essential sequence Ala-X2-X3-Ile-Glu). The amino acid substitution may be a conservative substitution. Examples of such conservative substitutions that naturally occur in amino acids include aliphatic amino acids (Gly, Ala, and Pro), hydrophobic amino acids (Ile, Leu, and Val), aromatic amino acids (Phe, Tyr, and Trp), acidic amino acids (Asp and Glu), basic amino acids (His, Lys, Arg, Gln, and Asn), and sulfur-containing amino acids (Cys and Met). The above amino acid deletion is located in a region not directly related to the activity of the decoy peptide of the present invention.

In one embodiment, X1 of Structural formula (I) of the present invention is 0 to 40, 0 to 30, 0 to 20, 0 to 13, 0 to 10, 0 to 3, or 0 to 1 amino acid residues.

In Structural formula (I), any amino acid that is not limited may also be located at X1. In one embodiment of the present invention, X1 consists of an amino acid sequence comprising 0 to 50, 0 to 40, 0 to 30, 0 to 20, 0 to 13, 0 to 10, 0 to 3, or 0 to 1 amino acid residues in the N-terminal direction of the 15th amino acid of the amino acid sequence of PLB.

In one embodiment, X1 comprises 0 or 1 amino acid residue in the N-terminal direction of the 15th amino acid of the amino acid sequence of PLB (SEQ ID NO: 27).

In one embodiment, in Structural formula (I) of the present invention, X4 is 0 to 50, 0 to 40, 0 to 33, 0 to 30, 0 to 20, 0 to 10, or 0 to 3 amino acid residues.

In Structural formula (I), any amino acid that is not limited may also be located at X4. In one embodiment of the present invention, X4 consists of an amino acid sequence comprising 0 to 50, 0 to 40, 0 to 33, 0 to 30, 0 to 20, 0 to 10, or 0 to 3 amino acid residues in the C-terminal direction of the 19th amino acid of the amino acid sequence of PLB (SEQ ID NO: 27).

In one embodiment, X4 comprises 0 to 3 amino acid residues in the C-terminal direction of the 19th amino acid of the amino acid sequence of PLB (SEQ ID NO: 27). In one embodiment, X4 comprises 0, 1, 2, or 3 amino acid residues in the C-terminal direction of the 19th amino acid of the amino acid sequence of PLB (SEQ ID NO: 27).

In one embodiment, X1 is Arg.

In one embodiment, X4 is Met, Met-Pro, or Met-Pro-Gln.

The 16th amino acid Ser residue (Ser16) and the 17th amino acid Thr residue (Thr17) of the amino acid sequence of the PLB of the present invention (SEQ ID NO: 27) are phosphorylation sites located in the flexible loop region of the PLB (the 14th to 22nd amino acid sequences of PLB). In Structural formula (I), X2 and X3 represent the amino acid positions where Ser16 and Thr17 are located within the PLB, respectively.

In the present invention, the decoy peptide of the present invention can be designed by substituting Ser16 and/or Thr17 with Glu or Asp. The decoy peptide comprising Glu or Asp at X2 and/or X3 in Structural formula (I) is similar to phosphorylated PLB and competes with the phosphorylation site of PLB for binding to PP1.

In one embodiment, X2 may be Glu or Asp, and X3 may be Thr or Glu. In another embodiment, X2 may be Glu or Asp, and X3 may be Thr.

In one embodiment, X1 may be Arg, X2 may be Glu, X3 may be Thr, and X4 may be Met-Pro-Gln (SEQ ID NO: 18); X1 may be Arg, X2 may be Ser, X3 may be Glu, and X4 may be Met-Pro-Gln (SEQ ID NO: 19); X1 may be Arg, X2 may be Asp, X3 may be Thr, and X4 may be Met-Pro-Gln (SEQ ID NO: 20); X2 may be Glu, X3 may be Thr, and X4 may be Met-Pro-Gln (SEQ ID NO: 21); X1 may be Arg, X2 may be Glu, X3 may be Thr, and X4 may be Met (SEQ ID NO: 22); or X1 may be Arg, X2 may be Glu, and X3 may be Thr (SEQ ID NO: 23).

In one embodiment, X1 may be Arg, X2 may be Glu, X3 may be Thr, and X4 may be Met-Pro; X1 may be Arg, X2 may be Asp, X3 may be Thr, and X4 may be Met; X1 may be Arg, X2 may be Asp, X3 may be Thr, and X4 may be Met-Pro; X1 may be Arg, X2 may be Asp, X3 may be Glu, and X4 may be Met; X1 may be Arg, X2 may be Asp, X3 may be Glu, and X4 may be Met-Pro; X1 may be Arg, X2 may be Asp, X3 may be Glu, and X4 may be Met-Pro-Gln; X1 may be Arg, X2 may be Glu, X3 may be Glu, and X4 may be Met; X1 may be Arg, X2 may be Glu, X3 may be Glu, and X4 may be Met-Pro; or X1 may be Arg, X2 may be Glu, X3 may be Glu, and X4 may be Met-Pro-Gln.

In one embodiment, the decoy peptide of the present invention comprises any one amino acid sequence selected from the group consisting of SEQ ID NOs: 18 to 23.

In one embodiment, the decoy peptide may comprise a sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to the amino acid sequence of SEQ ID NOs: 18 to 23.

In one embodiment, the nucleotide sequence encoding a decoy peptide may comprise the nucleotide sequence of SEQ ID NO: 7 or 9.

In one embodiment, the nucleotide sequence encoding a decoy peptide may comprise a sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to the nucleic acid sequence of SEQ ID NO: 7 or 9.

### CCN5 protein or fragment thereof

As used herein, the term "CCN5 protein (cellular communication network factor 5 protein)" refers to a stromal cell protein belonging to the CCN family that plays a variety of roles in regulating cellular functions, such as vascular diseases, angiogenesis, tumorigenesis, fibrotic disease induction, cell differentiation, and survival. The CCN5 protein, unlike other CCN family proteins, lacks a C-terminal domain and is also known as WISP-2, HICP, Cop1, CTGF-L, and the like. In addition, the CCN5 protein comprises a single polypeptide chain with a 250 amino acid sequence. The CCN5 protein has a 22-amino acid secretion induction sequence at its N-terminus, allowing it to be secreted to the outside of cells and function as a signaling protein.

Specifically, the CCN5 protein may comprise the amino acid sequence of SEQ ID NO: 26. In addition, the nucleotide encoding the CCN5 protein may comprise the nucleic acid sequence of SEQ ID NO: 13.

In one embodiment, the CCN5 protein may comprise an amino acid sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to SEQ ID NO: 26.

In one embodiment, the nucleotide encoding the CCN5 protein may comprise a nucleic acid sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to SEQ ID NO: 13.

In addition, the fragment of the CCN5 protein may be a fragment in which a portion of the N-terminus and/or C-terminus of the wild-type CCN5 is truncated, as long as the CCN5 protein activity is maintained. Specifically, the fragment of the CCN5 protein may be truncated by 1 to 30, 1 to 20, 1 to 10, or 1 to 5 amino acids from the N-terminus or C-terminus.

### Gene construct structure

As used herein, the term "gene construct" refers to a construct that enables the expression of a desired protein, etc., when recombined or introduced into a host strain or cell through transformation. It includes not only a gene encoding the desired protein, but also essential regulatory genes operably linked to enable the expression of the gene.

The gene construct may comprise a nucleotide encoding a decoy peptide and a nucleotide encoding a CCN5 protein or a fragment thereof, in a 5'- to 3'-end direction. In addition, the gene construct may comprise a nucleotide encoding a CCN5 protein or a fragment thereof and a nucleotide encoding a decoy peptide, in a 5'- to 3'-end direction. In addition, the gene construct may comprise a self-cleavage sequence or IRES sequence between the nucleotide encoding a decoy peptide and the nucleotide encoding a CCN5 protein or a fragment thereof.

As used herein, the term "self-cleavage sequence" refers to a protein that contains both a domain having a cleavage function and a recognition sequence that the above domain recognizes and cleaves, and when certain conditions are met, the cleavage function domain is activated to recognize and cleave the recognition sequence within the same protein. Specifically, the self-cleavage sequence may be a 2A sequence.

As used herein, the term "2A self-cleaving peptide" refers to a self-cleavage sequence found across a wide range of viral families, sharing the core sequence motif of DxExNPGP, and inhibiting ribosome formation of peptide bonds by inducing ribosome skipping during protein translation in cells.

At this time, the self-cleavage sequence may be a 2A peptide derived from porcine teschovirus-1 (PTV1) (P2A), Thosea asigna virus (TaV) (T2A), equine rhinitis A virus (ERAV) (E2A), or foot and mouth disease virus (FMDV) (F2A). Preferably, it may be P2A.

In one embodiment, the P2A may also have the amino acid sequence of SEQ ID NO: 24. In addition, the nucleotide encoding the P2A may be comprised of the nucleic acid sequence of SEQ ID NO: 11.

In one embodiment, the nucleotide encoding the P2A may comprise a nucleic acid sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to SEQ ID NO: 11.

In one embodiment, the T2A may have the amino acid sequence of SEQ ID NO: 39, the E2A may have the amino acid sequence of SEQ ID NO: 40, and the F2A may have the amino acid sequence of SEQ ID NO: 41.

As used herein, the term "IRES (internal ribosome entry site)" refers to an internal ribosome entry site or ribosome binding site that forms a loop structure on mRNA and initiates translation of the mRNA. If such an IRES is present, a gene located downstream of the IRES can be expressed. In one embodiment, the gene construct may comprise a nucleotide encoding a self-cleavage sequence between a nucleotide encoding a decoy peptide and a nucleotide encoding a CCN5 protein or a fragment thereof.

Specifically, the gene construct may comprise a nucleotide encoding a decoy peptide, a nucleotide encoding a self-cleavage sequence and a nucleotide encoding a CCN5 protein or a fragment thereof, in a 5'- to 3'-end direction.

### Cell-penetrating peptide

In addition, a nucleotide encoding a cell-penetrating peptide may be additionally linked to the gene construct. At this time, the cell-penetrating peptide may be additionally linked to the N-terminus and/or C-terminus.

As used herein, the term "cell-penetrating peptide (CPP)" refers to a short peptide that promotes cellular uptake and absorption, and is a peptide that can pass through the cell membrane of the phospholipid bilayer itself. The CPP can deliver a CPP-containing delivery agent into cells via direct penetration, endocytosis, or reverse micelle formation.

In order to transport the decoy peptide of the present invention into cardiomyocytes, the decoy peptide must comprise a cell-penetrating peptide. As used herein, the term cell-penetrating peptide refers to a peptide essential for transporting a specific peptide into cells, typically consisting of 10-50 amino acids or more.

At this time, the cell-penetrating peptide may be selected from the group consisting of a Tat-derived peptide, an arginine-rich peptide, a transportan, and an amphipathic peptide carrier. In addition, the cell-penetrating peptide may be a variety of antennapedia-based peptides or variants thereof having cell-penetrating properties, including retroinverso and D-isomer peptides (Brugidou, J. et.al., BiochemBiophys Res Commun., 214(2):685-93 (1995); Derossi, D. et.al., TrendsCellBiol., 8:84-87 (1998)). Preferably, the cell-penetrating peptide is a TAT peptide (Tat-derived peptide) or a variant thereof.

As used herein, the term "TAT peptide" refers to approximately 86 amino acids derived from the human immunodeficiency virus (HIV), also known as Tat-derived peptide, or TAT. The TAT peptide has a cysteine-rich, basic, and integrin-binding domain as their main protein domains. At this time, at least one amino acid can be substituted to enhance the cell penetration of the TAT peptide. In the present specification, the TAT peptide or a variant thereof is collectively referred to as "TAT peptide." For information on variants of the TAT peptide (Tat-derived peptides), see the full text of A novel cell-penetrating peptide to facilitate intercellular transport of fused proteins., Shen Y. *et.al., J Control Release.,* 2014 Aug 28.

In one embodiment, the TAT peptide of the present invention may have the amino acid sequence of SEQ ID NO: 16 or 38. In addition, the TAT peptide may comprise an amino acid sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to SEQ ID NO: 16 or 38.

As used herein, the term "TAT peptide variant" refers to a form in which a portion of an amino acid sequence in a full-length TAT peptide or a fragment thereof is substituted. That is, the TAT peptide may have an amino acid sequence different from that of the wild-type TAT peptide or a fragment thereof. However, the TAT peptide variant may have activity equivalent to or similar to that of the wild-type TAT peptide.

Specifically, the TAT peptide variant may be one in which a portion of an amino acid sequence in the TAT peptide is substituted. A specific example of a TAT peptide variant resulting from an amino acid substitution may be one in which at least one of the 2nd, 4th, 5th, 6th, 9th, and 11th amino acids in the amino acid sequence of SEQ ID NO: 38 is substituted. In one embodiment, one, two, three, four, five, six, seven, eight, nine, or ten amino acids may be substituted, as long as activity is maintained. In another embodiment, one to six amino acids may be substituted. In another embodiment, the TAT peptide variant may be one in which the 2nd, 4th, 5th, 6th, 9th, and 11th amino acids in the amino acid sequence of SEQ ID NO: 38 are substituted.

At this time, the "other amino acid" introduced by the substitution may be any one selected from the group consisting of alanine (Ala, A), arginine (Arg, R), asparagine (Asn, N), aspartic acid (Asp, D), cysteine (Cys, C), glutamic acid (Glu, E), glutamine (Gln, Q), histidine (His, H), isoleucine (Ile, I), leucine (Leu, L), lysine (Lys, K), methionine (Met, M), phenylalanine (Phe, F), glycine (Gly, G), proline (Pro, P), serine (Ser, S), threonine (Thr, T), tryptophan (Trp, W), tyrosine (Tyr, Y), and valine (Val, V). However, in the amino acid substitution of the variant, the second amino acid in the amino acid sequence of SEQ ID NO: 38 cannot be substituted with glycine (G), the fourth and fifth amino acids cannot be substituted with lysine (K), and the sixth, ninth, and eleventh amino acids cannot be substituted with arginine (R).

In one embodiment, the second amino acid in the amino acid sequence of SEQ ID NO: 38 may be substituted with an amino acid other than glycine (G); the fourth and fifth amino acids may be substituted with an amino acid other than lysine (K); and the sixth, ninth, and eleventh amino acids may be substituted with an amino acid other than arginine (R).

In one embodiment, the second glycine (G) in the amino acid sequence of SEQ ID NO: 38 may be substituted with alanine (A). In the amino acid sequence of SEQ ID NO: 38, the 4th and 5th lysines (K) may be substituted with alanine (A). In the amino acid sequence of SEQ ID NO: 38, the 6th, 9th, and 11th arginines (R) may be substituted with alanine (A).

Specifically, the TAT peptide variant may have at least one substitution selected from the group consisting of G2A, K4A, K5A, R6A, R9A, and R11A in the amino acid sequence of SEQ ID NO: 38.

Specifically, the TAT peptide variant may have two, three, four, five, or six amino acid substitutions at positions selected from G2A, K4A, K5A, R6A, R9A, and R11A in the amino acid sequence of SEQ ID NO: 38. Furthermore, the variant may have G2A, K4A, K5A, R6A, R9A, and R11A mutations in the amino acid sequence of SEQ ID NO: 38.

Specifically, the TAT peptide variant may comprise the amino acid sequence of SEQ ID NO: 16.

In addition, the TAT peptide variant may comprise a repeat of the amino acid sequence of SEQ ID NO: 16. The amino acid sequence of SEQ ID NO: 16 may be repeated 1 to 5 times. Specifically, it may be repeated 1 to 4 times, or 1 to 3 times, and preferably, it may be repeated 3 times. The repeat may be directly linked without a peptide linker and may be repeated.

In one embodiment, the TAT peptide variant may have the amino acid sequence of SEQ ID NO: 15.

In addition, the nucleotide sequence encoding the TAT peptide or a variant thereof of the present invention may comprise the nucleic acid sequence of SEQ ID NO: 2. In addition, the nucleotide encoding the TAT peptide may comprise a nucleic acid sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to SEQ ID NO: 2.

In one embodiment, the gene construct may comprise a nucleotide encoding a cell-penetrating peptide and a nucleotide encoding a decoy peptide, in a 5'- to 3'-end direction. In addition, the gene construct may comprise a nucleotide encoding a decoy peptide and a nucleotide encoding a cell-penetrating peptide, in a 5'- to 3'-end direction.

In addition, the gene construct may comprise multiple the cell-penetrating peptides, including one, two, or three. In addition, when multiple cell-penetrating peptides are included, they may be located at the 5'-end and/or the 3'-end of the nucleotide encoding the decoy peptide.

### Embodiments of gene construct structure

In one embodiment, the gene construct may comprise a nucleotide encoding a cell-penetrating peptide, a nucleotide encoding a decoy peptide, and a nucleotide encoding a CCN5 protein or a fragment thereof, in a 5'- to 3'-end direction.

In one embodiment, the gene construct may comprise a nucleotide encoding a cell-penetrating peptide, a nucleotide encoding a decoy peptide, a nucleotide encoding a self-cleavage sequence, and a nucleotide encoding a CCN5 protein or a fragment thereof, in a 5'- to 3'-end direction.

In one embodiment, it was confirmed that when SP-TATm3-SE(decoy peptide)-P2A-CCN5, which is a specific example of the gene construct, is expressed in a cell, the decoy peptide and the CCN5 protein can each be secreted to the outside of cells. Furthermore, it was confirmed that the secreted decoy peptide and CCN5 can re-enter the cells. Therefore, it was confirmed that when SP-TATm3-SE(decoy peptide)-P2A-CCN5 is expressed in a subject, the decoy peptide and CCN5 can be secreted from the cells in which they are produced and absorbed into surrounding cells and tissues.

The gene construct may further comprise the nucleotide encoding a decoy peptide. That is, the gene construct may comprise multiple nucleotides encoding a decoy peptide. For example, the genetic construct may comprise 1 to 7, 1 to 6, 2 to 6, 2 to 5, 1 to 3, or 2 to 3 nucleotides encoding a decoy peptide.

In one embodiment, when the genetic construct comprises nucleotides encoding a decoy peptide in duplicate, each decoy peptide may be sequentially linked by a linker. At this time, the linker may be a peptide linker.

In one embodiment, the gene construct may consist of the following Structural formula (II):

[ SP1 - X - Y - ((L1)p - Y)n- (L2)q - Z ]m - SP2 - T (II)

wherein, in Structural formula (II),
X is the nucleotide encoding a cell-penetrating peptide,
Y is the nucleotide encoding a decoy peptide,
SP1 and SP2 are nucleotides encoding a signal sequence,
L1 and L2 are peptide linkers,
Z is a nucleotide encoding the cleavage sequence,
T is the nucleotide encoding a CCN5 protein or a fragment thereof,
n is any one integer from 0 to 3,
m is any one integer from 1 to 3, and
p and q are each 0 or 1.

The cell-penetrating peptide, decoy peptide, CCN5 protein, or fragment thereof is the same as described above.

The signal sequence refers to a signal peptide (SP), and may be positioned in front of the sequence encoding the peptide to expose the decoy peptide and the CCN5 protein to the outside of the cell membrane, i.e., for extracellular secretion.

In one embodiment, the signal peptide (SP1) may comprise the amino acid sequence represented by SEQ ID NO: 14, and the base sequence encoding it may be represented by SEQ ID NO: 1. In addition, the signal peptide (SP2) may comprise the amino acid sequence represented by SEQ ID NO: 25, and the base sequence encoding it may be represented by SEQ ID NO: 12.

The peptide linker may comprise 1 to 30 consecutive amino acids, 1 to 20 consecutive amino acids, or 1 to 15 consecutive amino acids, or may be comprised of 1 to 10 amino acids. In one embodiment, the peptide linker may comprise 1 to 8 amino acids or 1 to 5 amino acids.

In one embodiment, the linker (L) may be a glycine linker, a peptide linker comprised of the amino acid sequence of SEQ ID NO: 17, or a peptide linker encoded by the nucleotide sequence of SEQ ID NO: 6.

In one specific example, when n is 0, m is 1, p is 0, and q is 0, the gene construct may have the structure of SP-X-Y-Z-SP-T in a 5'- to 3'-end direction. At this time, one specific example of the gene construct may have the structure of SP1-TATm3-SE-P2A-SP2-CCNS.

In one specific example, when n is 1, m is 1, p is 1, and q is 0, the gene construct may have the structure SP-X-Y-L-Y-Z-SP-T in a 5'- to 3'-end direction. At this time, one specific example of the gene construct may have the structure of SP1-TATm3-SE-ggg-SE-P2A-SP2-CCN5.

In one specific example, when n is 2, m is 1, p is 1, and q is 0, the gene construct may have the structure SP-X-Y-L-Y-L-Y-Z-SP-T in a 5'- to 3'-end direction. At this time, one specific example of the gene construct may have the structure of SP1-TATm3-SE-ggg-SE-ggg-SE-P2A-SP2-CCN5.

In one specific example, when n is 1, m is 2, p is 1, and q is 0, the gene construct may have the structure of SP-X-Y-L-Y-Z-SP-X-Y-L-Y-Z-SP-T in the 5'-to-3'-end direction. At this time, one specific example of the gene construct may have the structure of SP1-TATm3-SE-ggg-SE-P2A-SP1-TATm3-SE-ggg-SE-P2A-SP2-CCN5.

In one specific example, when n is 0, m is 2, p is 0, and q is 0, the gene construct may have the structure of SP-X-Y-Z-SP-X-Y-Z-SP-T in a 5'- to 3'-end direction. At this time, one specific example of the gene construct may have the structure of SP1-TATm3-SE-P2A-SP1-TATm3-SE-P2A-SP2-CCN5.

In one specific example, when n is 2, m is 2, p is 1, and q is 0, the gene construct may have the structure of SP-X-Y-L-Y-L-Y-Z-SP-X-Y-L-Y-L-Y-Z-SP-T in a 5'- to 3'-end direction. At this time, one specific example of the gene construct may have the structure of SP1-TATm3-SE-ggg-SE-ggg-SE-P2A-SP1-TATm3-SE-ggg-SE-ggg-SE-P2A-SP2-CCN5.

### Recombinant vector comprising gene construct

In another aspect of the present invention, there is provided a recombinant vector comprising the gene construct.

As used herein, the term " recombinant vector" refers to a recombinant expression vector capable of expressing a target protein in a target host cell, and refers to a gene construct that comprises essential regulatory elements operatively linked to express the gene insert.

In addition, the recombinant expression vector may comprise a signal sequence for export of the fusion polypeptide to facilitate protein isolation from cell culture solution. A specific initiation signal may also be required for efficient translation of the inserted nucleic acid sequence. These signals include the ATG start codon and adjacent sequences. In some cases, an exogenous translation control signal, possibly including an ATG start codon, must be provided. These exogenous translation control signals and start codons can be from a variety of natural and synthetic sources. Expression efficiency can be increased by introducing appropriate transcription or translation enhancing factors.

The recombinant vector of the present invention is a means for introducing into a cell and expressing a protein, and any known recombinant vector, such as a plasmid vector, a cosmid vector, or a bacteriophage vector, can be used. The recombinant vector can be easily produced by those of ordinary skill in the art according to any known method using DNA recombination technology.

For example, pWE15, M13, Charon4A, and Charon21A can be used as phage or cosmid vectors, and pBR, pUC, pBluescriptII, pGEM, pTZ, pCL, and pET can be used as plasmid vectors. The recombinant vector usable in the present invention is not particularly limited, and any known expression vector can be used.

Methods for introducing the recombinant vector into a host cell may be any method known in the art. For example, but not limited to, transient transfection, microinjection, transduction, cell fusion, calcium phosphate precipitation, liposome-mediated transfection, DEAE dextran-mediated transfection, polybrene-mediated transfection, electroporation, gene gun, or other known methods for introducing nucleic acids into cells can be used (Wu et al., J. Bio. Chem., 267:963-967, 1992; Wu and Wu, J. Bio. Chem., 263:14621-14624, 1988).

### Recombinant virus comprising gene construct

In another aspect of the present invention, there is provided a recombinant virus comprising the gene construct.

The virus may be any one selected from the group consisting of adenovirus, adeno-associated virus (AAV), retrovirus, lentivirus, herpes simplex virus, anellovirus (Anelloviridae), and vaccinia virus. Specifically, the virus may be adeno-associated virus.

The adenoviruses are widely used as a gene delivery vector due to their moderate genome size, ease of manipulation, high titer, broad target cell range, and excellent infectivity. Both ends of the genome can comprise 100 to 200 bp inverted terminal repeats (ITRs), which are cis elements essential for DNA replication and packaging. The genome may further comprise the E1 region (E1A and E1B), which encodes proteins involved in viral DNA replication.

However, among adenovirus vectors, replication-deficient adenoviruses lacking the E1 region can be used. Meanwhile, the E3 region is removed from conventional adenovirus vectors, providing a site for insertion of foreign genes.

These retroviruses are widely used as a gene delivery vector because they can insert their genes into the host genome, carry large amounts of foreign genetic material, and have a wide spectrum of cells they can infect. In order to construct a retroviral vector, a genetic construct comprising the decoy gene and the CCN5 gene may be inserted into the retroviral genome in place of the retroviral sequence, resulting in the production of a replication-deficient virus.

The adeno-associated virus (AAV) is suitable as a gene delivery system of the present invention because it can infect non-dividing cells and has the ability to infect various cell types. An AAV virus having a gene construct of the present invention can be produced by cotransfecting a plasmid comprising a gene construct comprising SE and CCN5 genes flanked by two AAV terminal repeats and an expression plasmid comprising a wild-type AAV coding sequence without terminal repeats.

The vector derived from vaccinia virus, lentivirus, or herpes simplex virus can also be used to deliver the gene for the CCN5 protein and the desired nucleotide sequence to be delivered into cells.

### Pharmaceutical composition

In another aspect of the present invention, there is provided a pharmaceutical composition comprising the gene construct, the recombinant vector, or the recombinant virus as an active ingredient.

The pharmaceutical composition may be for the prevention or treatment of a heart disease.

The term "heart disease" refers to all diseases occurring in the heart, such as those resulting from weakened cardiac function. The heart disease may be selected from the group consisting of heart failure, ischemia, arrhythmia, myocardial infarction, congestive heart failure, transplant rejection, abnormal cardiac contraction, and abnormal Ca²⁺ metabolism, but is not limited thereto. Preferably, it may be heart failure.

Pharmaceutically acceptable carriers included in the pharmaceutical composition of the present invention, when in the form of a formulation, may include at least one selected from the group consisting of lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methyl hydroxybenzoate, propyl hydroxybenzoate, talc, magnesium stearate, and mineral oil, but are not limited thereto.

The dosage form of the pharmaceutical composition may vary depending on the method of use, but may be prepared as an injection.

The dosage of the pharmaceutical composition of the present invention is preferably determined in consideration of the patient's age, sex, condition, absorption rate of the active ingredient in the body, inactivation rate, and concomitantly administered drugs. When the pharmaceutical composition is a virus, it can be administered in an amount of 1.0×10³ to 1.0×10²⁰ viral genomes per day for an adult. Specifically, the dosage of the pharmaceutical composition of the present invention can be administered in an amount of 1.0×10³ to 1.0×10²⁰, 1.0×10⁸ to 1.0×10¹⁶, 1.0×10¹² to 1.0×10¹⁵, or 1.0×10¹³ to 1.0×10¹⁴ per day for an adult.

### Pharmaceutical composition for combination administration

In another aspect of the present invention, there is provided a pharmaceutical composition for preventing or treating heart failure, comprising the decoy peptide and the CCN5 protein as an active ingredient.

The pharmaceutical composition of the present invention is administered parenterally, and parenteral administration includes intravenous injection, subcutaneous injection, intramuscular injection, intraperitoneal injection, transdermal administration, inhalation administration using the nasal cavity, direct injection into tissue, and the like.

As used herein, the term "acceptable carrier" includes any or all of the following materials, including any solvent, diluent, liquid transporter, dispersant, suspending aid, surfactant, isotonic agent, thickener, emulsifier, preservative, solid binder, lubricant, or similar agents suitable for a particular dosage. Examples of pharmaceutical compositions of pharmaceutically acceptable carriers include, but are not limited to: Sugars such as glucose and sucrose; starches such as corn starch and potato starch; cellulose and its derivatives such as sodium carboxymethylcellulose, ethylcellulose, and cellulose acetate; powdered tragacanth; malt; gelatin; talc; excipients such as cocoa butter, suppository wax, peanut butter, cottonseed oil, safflower oil, sesame oil, olive oil, and soybean oil; glycols such as propylene glycol; esters such as ethyl oleate and ethyl laurate; agar; buffering agents such as magnesium hydroxide and aluminum hydroxide; alginic acid; pyrogen-free distilled water; isotonic saline; Ringer's solution; ethyl alcohol and phosphate-buffered water, and sodium lauryl sulfate and magnesium stearate, as well as coloring agents, colorants, release agents, coating agents, sweeteners, flavoring agents, fragrances, and antioxidants may be included at the discretion of the compound manufacturer.

### Use of pharmaceutical composition

In another aspect of the present invention, there is provided a method for preventing or treating a heart disease, comprising administering the pharmaceutical composition to a subject.

As described above, the pharmaceutical composition may be a pharmaceutical composition for preventing or treating a heart disease, comprising a gene construct comprising a nucleotide encoding a decoy peptide that inhibits protein phosphatase 1 (PP1)-mediated dephosphorylation of phospholamban (PLB); and a nucleotide encoding a CCN5 protein or a fragment thereof as an active ingredient.

In addition, the pharmaceutical composition may be a pharmaceutical composition for preventing or treating a heart disease, comprising a recombinant vector comprising the gene construct as an active ingredient.

In addition, the pharmaceutical composition may be a pharmaceutical composition for preventing or treating a heart disease, comprising a recombinant virus comprising the gene construct as an active ingredient.

The subject may be a mammal, and preferably, may be a human. Specifically, the subject may be a human or non-human mammal that may be suffering from or is suffering from a heart disease.

In another aspect of the present invention, there is provided a pharmaceutical composition for preventing or treating a heart disease, comprising a recombinant virus comprising a gene construct comprising a nucleotide encoding a decoy peptide; and a recombinant virus comprising a gene construct comprising a nucleotide encoding a CCN5 protein or a fragment thereof.

The decoy peptide is the same as described above in the gene construct. The CCN5 protein or a fragment thereof is the same as described above in the gene construct.

In another aspect of the present invention, there is provided a use of the pharmaceutical composition for the prevention or treatment of a heart disease.

In another aspect of the present invention, there is provided a use of the pharmaceutical composition for the manufacture of a medicament for preventing or treating a heart disease.

### Mode for Carrying out the Invention

Hereinafter, the present invention will be described in detail through preparation examples and examples. However, the following preparation examples and examples are only intended to illustrate the present invention, and the present invention is not limited to the following preparation examples and examples.

### Preparation Example 1. Production of gene construct

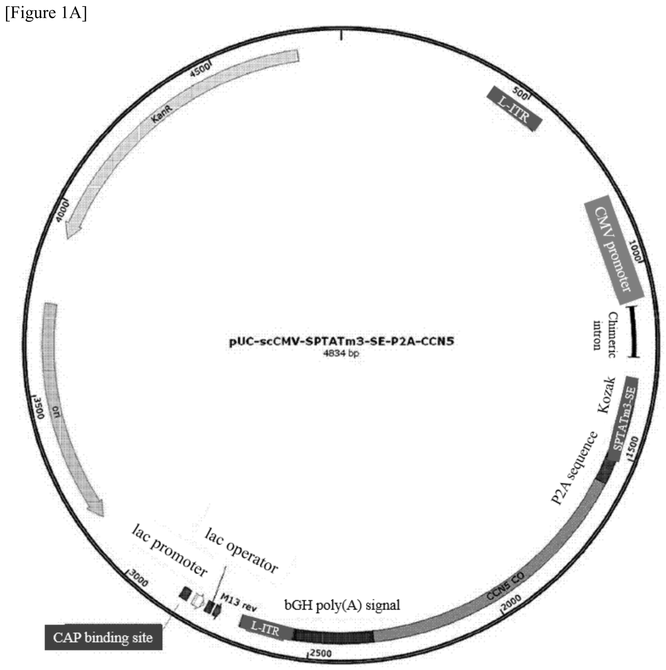

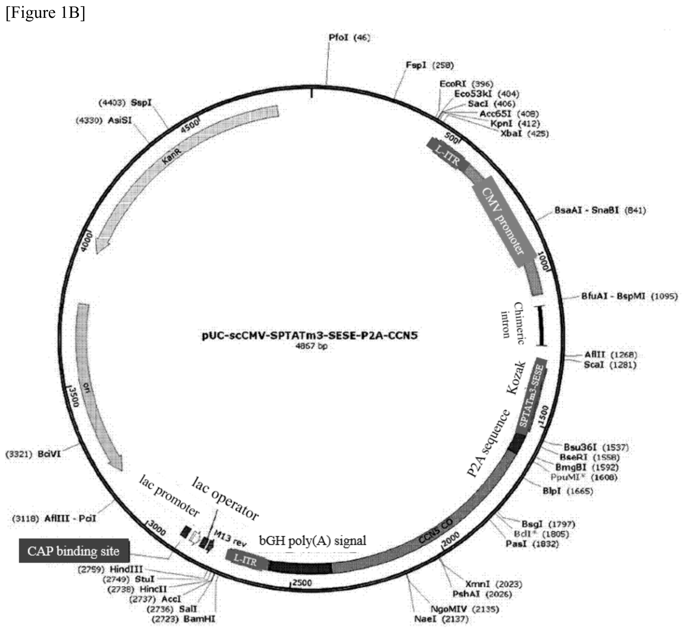

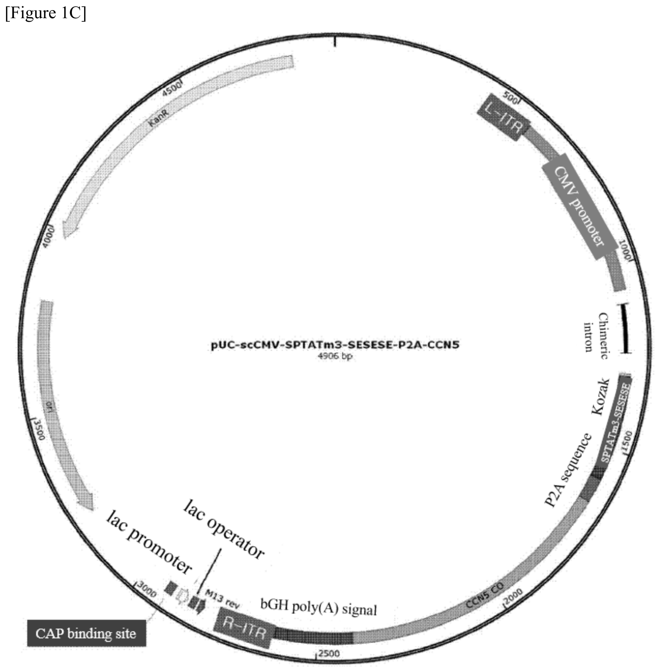

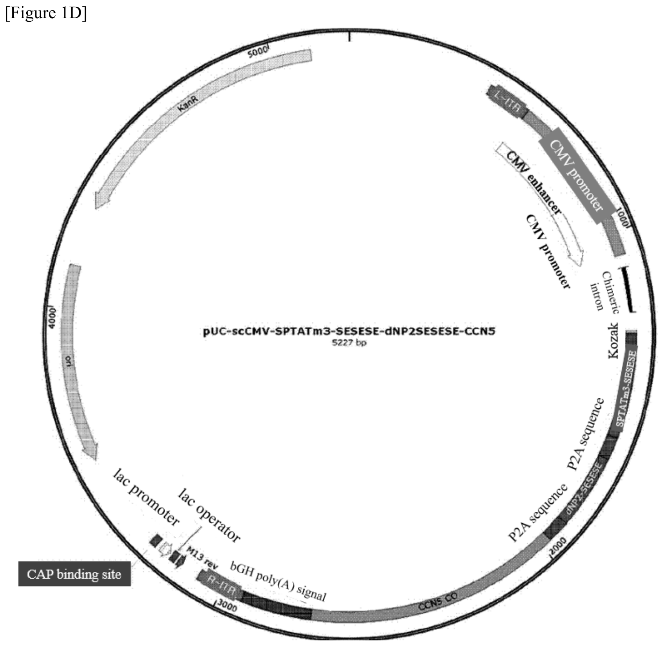

In order to express the CCN5 protein and the SE peptide (decoy peptide) separately and simultaneously, the following gene constructs were produced, as shown in Figures 1A to 1D: pUC-scCMV-SPTATm3-SE-P2A-CCN5, pUC-scCMV-SPTATm3-SESE-P2A-CCN5, pUC-scCMV-SPTATm3-SESESE-P2A-CCNS5, pUC-scCMV-SPTATm3-SESESE-P2A-CCN5, and pUC-scCMV-SPTATm3-SESESE-dNP2-SESESE-CCN5.

As shown in Table 1 below, they were produced in a form including a nucleotide sequence encoding a signal peptide (SP) for secreting the peptide to the outside of cells, a cell-penetrating peptide (CPP) TATm3, and a nucleotide sequence encoding an SE peptide (decoy peptide). The self-cleavage sequence P2A was then linked, followed by the CCN5 protein. The glycine linkers (ggg) were used to connect between the CPP and the SE peptide, between the repeated SE peptides, and between the SE peptide and the P2A sequences.

**[Table 1]**

| | Nucleotide sequence |
|---|---|
| Signal peptide 1 ( SEQ ID NO: 1 ) | ATGTGGTGGCGCCTTTGGTGGCTCCTTTTGTTGCTG TTGTTGTTGTGGCCTATGGTGTGGGCCAAGCTG |
| TATm3(CPP) ( SEQ ID NO: 2 ) | TATGCAAGAGCCGCTGCAAGACAGGCCAGAGCATA TGCGAGGGCAGCTGCTAGACAGGCTAGAGCTTATG CTAGAGCAGCTGCCAGGCAGGCTAGAGCT |
| Glycine X3 linker 1 ( SEQ ID NO: 6 ) | GGTGGAGGA |
| SE(Decoy peptide) ( SEQ ID NO: 7 ) | AGAGCCGAGACCATCGAAATGCCACAA |
| Glycine X3 linker 2 ( SEQ ID NO: 8 ) | GGTGGCGGA |
| SE(Decoy peptide) ( SEQ ID NO: 9 ) | CGGGCCGAGACAATAGAAATGCCTCAG |
| Glycine X3 linker 3 ( SEQ ID NO: 10 ) | GGAGGAGGA |
| P2A sequence ( SEQ ID NO: 11 ) | GGAAGCGGCGCTACTAACTTCTCCCTCCTTAAACA AGCCGGAGACGTGGAAGAGAATCCAGGTCCC |
| Signal peptide 2 ( SEQ ID NO: 12 ) | ATGCGTGGTACTCCTAAAACCCATCTGCTGGCTTTT TCTCTGCTGTGTCTGCTGAGCAAAGTTCGTACT |
| CCN5 w/o signal pept ide ( SEQ ID NO: 13 ) | CAGCTGTGCCCGACCCCGTGTACTTGTCCATGGCCA CCACCGCGTTGTCCTCTGGGTGTACCTCTGGTTCTG GACGGTTGCGGCTGTTGTCGTGTTTGCGCCCGTCGT CTGGGCGAACCTTGTGATCAGCTGCACGTTTGCGAC GCTTCCCAGGGTCTGGTTTGCCAGCCTGGTGCTGGT CCTGGTGGTCGTGGTGCACTGTGCCTGCTGGCTGAA GACGACTCTTCTTGTGAAGTAAACGGTCGTCTGTAC CGTGAAGGTGAGACCTTCCAGCCACATTGCTCTATT CGCTGCCGCTGCGAAGATGGCGGCTTTACTTGTGTT CCTCTGTGCTCCGAAGACGTTCGTCTGCCGTCTTGG GATTGTCCTCATCCGCGTCGTGTTGAAGTGCTGGGC AAATGCTGCCCGGAATGGGTTTGCGGTCAAGGTGG TGGTCTGGGTACTCAACCGCTGCCGGCTCAGGGTC CACAATTCTCTGGCCTGGTTAGCAGCCTGCCGCCTG GTGTTCCTTGTCCAGAATGGAGCACGGCTTGGGGC CCTTGTTCTACCACTTGCGGCCTGGGTATGGCTACC CGTGTTTCCAACCAGAACCGTTTTTGTCGTCTGGAA ACCCAGCGTCGTCTGTGTCTGAGCCGTCCGTGTCCG CCATCTCGTGGTCGTTCCCCACAGAACTCCGCTTTC TGA |

At this time, the P2A sequence was a self-cleavage site derived from porcine teschovirus-1 and was composed of a base sequence encoding 22 amino acids, and CCN5 was a codon-optimized sequence of the human CCN5 protein.

The amino acid sequences expressed using the above gene constructs are shown in Table 2 below.

**[Table 2]**

| | Amino acid sequence |
|---|---|
| Signal peptide 1 ( SEQ ID NO: 14 ) | MWWRLWWLLLLLLLLWPMVWAKL |
| TATm3(CPP) ( SEQ ID NO: 15 ) | YARAAARQARAYARAAARQARAYARAAARQARA |
| Glycine X3 linker 1 ( SEQ ID NO: 17 ) | GGG |
| SE1(Decoy peptide) ( SEQ ID NO: 18 ) | RAETIEMPQ |
| Glycine X3 linker 2 ( SEQ ID NO: 17 ) | GGG |
| SEI (Decoy peptide) ( SEQ ID NO: 18 ) | RAETIEMPQ |
| Glycine X3 linker 3 ( SEQ ID NO: 17 ) | GGG |
| P2A sequence ( SEQ ID NO: 24 ) | GSGATNFSLLKQAGDVEENPGP |
| Signal peptide 2 ( SEQ ID NO: 25 ) | MRGTPKTHLLAFSLLCLLSKVRT |
| CCN5 w/o signal pept ide ( SEQ ID NO: 26 ) | QLCPTPCTCPWPPPRCPLGVPLVLDGCGCCRVCARRL GEPCDQLHVCDASQGLVCQPGAGPGGRGALCLLAED DSSCEVNGRLYREGETFQPHCSIRCRCEDGGFTCVPL CSEDVRLPSWDCPHPRRVEVLGKCCPEWVCGQGGG LGTQPLPAQGPQFSGLVSSLPPGVPCPEWSTAWGPCS TTCGLGMATRVSNQNRFCRLETQRRLCLSRPCPPSRG RSPQNSAF |

### Preparation Example 2. Production of AAV vector

AAV vectors were produced for the structures BTG001 (expressing CCN5 only), BTG010 (expressing CPP and decoy peptide (TATm3-SESE)), and BTG011 (expressing TATm3-SESE and CCN5). The three AAV viruses, BTG001, BTG010, and BTG011, are as follows:
BTG001 is an adeno-associated virus that packages a construct that exclusively expresses the CCN5 protein via the CMV promoter. During gene delivery, BTG001 expresses the CCN5 protein in target tissues according to the tropism of AAV, and the CCN5 protein is secreted from infected cells into the extracellular matrix for its function.

BTG010 is an adeno-associated virus that packages a construct that exclusively expresses the SPTATm3-SESE peptide via the CMV promoter. During gene delivery, BTG010 expresses the SPTATm3-SESE peptide in target tissues according to the tropism of AAV, and the SPTATm3 is secreted from infected cells into the extracellular matrix for its function.

BTG011 is an adeno-associated virus that packages a construct that simultaneously expresses the SPTATm3-SESE peptide and the CCN5 protein via the CMV promoter. Due to the self-cleavage sequence between SPTATm3-SESE and CCN5, BTG011 expresses both the peptide and the protein, respectively, and exerts its effects on target tissues according to the tropism of AAV. In addition, SPTATm3-SESE and CCN5 are secreted from infected cells into the extracellular matrix for their function.

Specifically, the gene constructs ITR-CMV-SPTATm3-SESE-bGH poly(A)-ITR, ITR-CMV-CCN5-bGH poly(A)-ITR, and ITR-CMV-SPTATm3-SESE-P2A-CCN5-bGH poly(A)-ITR were cloned into the pUC57-kan vector, respectively, to produce the recombinant plasmids pUC-scCMV-CCN5 (BTG001), pUC-scCMV-SPTATm3-SESE (BTG010), and pUC-scCMV-SPTATm3-SESE-P2A-CCN5 (BTG011).

The protein produced by BTG011, one of the above recombinant plasmids, is cleaved into the SPTATm3-SESE peptide and the CCN5 protein by self-cleavage between the 21st amino acid (glycine) and the 22nd amino acid (proline) in the P2A region. The SPTATm3-SESE peptide and the CCN5 protein are transported into the endoplasmic reticulum and secreted into the extracellular space in a form in which the signal peptide is cleaved, enabling them to perform their intended functions.

The self-complementary adeno-associated virus (AAV, serotype 9) was produced using 293T cells. pUC-scCMV-CCN5 (BTG001), pUC-scCMV-SPTATm3-SESE (BTG010), and pUC-scCMV-SPTATm3-SESE-P2A-CCN5 (BTG011) were transfected into cells simultaneously with the Rep-Cap plasmid and helper plasmid required for virus packaging to produce BTG001, BTG010, and BTG011, respectively.

The AAV particles in the cell culture solution were collected, precipitated with ammonium sulfate, and purified by ultracentrifugation using an iodixanol gradient. The AAV particles were concentrated through multiple dilution and concentration steps, using centrifugation to exchange iodixanol for lactate Ringer's solution. The AAV concentration was quantified using quantitative RT-PCR and SDS-PAGE.

### Example 1. Transfection and conditioned medium treatment

3 µg of the plasmid produced in Preparation Example 1 was mixed with lipofectamine 2000 (Invitrogen) at a 1:2 (w/v) ratio in AD293 cells. The mixture was incubated for 30 minutes, and the cells were treated therewith. The medium was replaced after 4 hours, and the medium was harvested 24 hours later and the cells were treated with the medium for each experimental group.

### Example 2. Western blot

The cells or cardiac tissues were homogenized in a minimal volume of 50 mM Tris-HCl, pH 7.4, supplemented with Broad Spectrum Protease Inhibitor Cocktail (Calbiochem). The proteins were isolated by SDS-PAGE and transferred to polyvinylidene fluoride membranes (Schleicher & Schuell). After blocking with 5% (w/v) skim milk for 1 hour and washing with TBST, the membranes were reacted with indicated antibodies. The antibodies used in the present invention are as follows: CCN5 antibody (Sigma Aldrich), HA (Roche), α-SMA (Sigma Aldrich), p21 (Invitrogen), myc (Santa Cruz), PCNA (Abcam), phospho-eNOS (ser1177) (Cell signaling), eNOS (Cell signaling), and GAPDH antibody (Sigma-Aldrich). The membrane was then reacted with HRP-conjugated secondary antibody (Jackson ImmunoResearch, WestGrove, PA, USA) and developed using a chemiluminescent substrate (ECL western solution, DoGen, Korea). Images were captured and quantified using LAS software.

### Example 3. Quantitative RT-PCR

Real-time PCR was performed using the QuantiTect SYRB Green real time PCR Kit (Qiagen Ltd) to analyze transcription levels. RNA was isolated from cardiac tissue using Trizol (gibco BRL), and cDNA was synthesized. Various quantitative real time PCR conditions were 37 cycles: 94°C for 10 s, 57°C for 15 s, and 72°C for 5 s. The primers used in the experiments are listed in Table 3 below.

**[Table 3]**

| | Forward | Reverse |
|---|---|---|
| Human α-SMA | 5'-GCCCAGCCAAGCACTGTCA GGA-3' （SEQ ID NO: 29） | 5'-TCCCACCATCACCCCCTG ATGTC-3' (SEQ ID NO: 30) |
| Human Fibronectin | 5'-CTGGCCGAAAATACATTGT AAA-3' (SEQ ID NO: 31) | 5'-CCACAGTCGGGTCAGGA G-3' (SEQ ID NO: 32) |
| Human Vimentin | 5'-GAGAACTTTGCCGTTGAAG C-3' （SEQ ID NO: 33） | 5'-CGTGATGCTGAGAAGTT TCG-3' (SEQ ID NO: 34) |
| 18s rRNA | 5'-TAACGAACGAGACTCTGGC AT-3' ( SEQ ID NO: 36 ) | 5'-CGGACATCTAAGGGCAT CACAG-3' ( SEQ ID NO: 37 ) |

Data are expressed as mean ± SD value. Group means were compared using Student's test or one-way ANOVA with Bonferroni's post-hoc test (Statview, V5.0, SAS). P < 0.05 was considered statistically significant.

### Example 4. Confirmation of gene construct expression

### Example 4.1. Confirmation of extracellular secretion of SE peptide and CCN5 by SPTATm3

AD293 cells were transfected with 3 µg of the DNA plasmid constructs CCN5, SE-CCN5, SPTATm3-SE-CCN5, and dNP2-SE-CCN5. After 24 hours, the expression of the SE peptide and the CCN5 protein in intracellular protein lysates and media was confirmed by Western blotting. At this time, the expression of the SE peptide was confirmed using HA and myc tags.

At this time, the structures of CCN5, SE-CCN5, SPTATm3-SE-CCN5, and dNP2-SE-CCN5 were pds-hCCN5, pcDNA3.1(+)-SEgggSEgggSE-HA-P2A-SEgggSEgggSE-myc-P2A-CCN5, pcDNA3.1(+)-SPTATmX3-SEgggSEgggSE-HA-P2A-SPTATmX3-SEgggSEgggSE-myc-P2A-CCN5, and pcDNA3.1(+)-dNP2-SEgggSEgggSE-HA-P2A-dNP2-SEgggSEgggSE-myc-P2A-CCN5, respectively, in that order.

As a result, as shown in Figures 2 and 3, it was confirmed that the construct using SPTATm3 as a CPP secreted the SE peptide and CCN5 to the outside of cells more effectively than the construct using dNP2 as a CPP or the construct without CPP.

### Example 4.2. Confirmation of CCN5 expression level depending on repeats of SE peptides

AD293 cells were transfected with 3 µg of the DNA plasmid constructs SPTATm3-SE-CCN5, SPTATm3-SESE-CCN5, SPTATm3-SESESE-CCN5, and SPTATm3-dNP2SE-CCN5. After 24 hours, the expression of the SE peptide and the CCN5 protein in the media was confirmed by Western blotting. At this time, the expression of the SE peptide was confirmed using HA and myc tags.

At this time, the structures of the constructs SPTATm3-SE-CCN5, SPTATm3-SESE-CCN5, SPTATm3-SESESE-CCN5, and SPTATm3-dNP2SE-CCN5 were SPTATmX3-SE-HA-P2A-CCN5, SPTATmX3-SEgggSE-HA-P2A-CCN5, SPTATmX3-SEgggSEgggSE-HA-P2A-CCN5, and SPTATmX3-SEgggSEgggSE-HA-dNP2-SEgggSEgggSE-myc-P2A-CCN5, respectively, in that order.

As a result, as shown in Figure 4, it was confirmed that the construct using only SPTATm3 as a CPP showed higher extracellular expression levels of the SE peptide and CCN5 than the construct using both SPTATm3 and dNP2 as CPPs, and all constructs containing one to three repeats of the SE peptide showed high expression levels of the SE peptide and CCN5.

### Example 4.3. Confirmation of introduction of SE peptide and CCN5 into various types of cells

AD293 cells were transfected with 3 µg of the plasmid constructs SE-CCN5, SESE-CCN5, SESESE-CCN5, and CCN5 DNA, and the medium was collected 24 hours later. The collected medium was applied to smooth muscle cells (SMCs), endothelial cells (ECs), and normal human ventricular cardiac fibroblasts (NHCF-v), respectively.

At this time, the structures of the constructs SE-CCN5, SESE-CCN5, SESESE-CCN5, and CCN5 were SPTATmX3-SE-HA-P2A-CCN5, SPTATmX3-SEgggSE-HA-P2A-CCN5, SPTATmX3-SEgggSEgggSE-HA-P2A-CCN5, and CCN5-only, respectively, in that order.

Western blotting was then performed to confirm the presence of the SE peptide and the CCN5 protein in each cell. Specifically, we confirmed whether the SE peptide and the CCN5 protein were successfully introduced into each cell.

As a result, as shown in Figures 5 and 6, it was confirmed that the constructs with two repeats of the SE peptide showed high expression levels of the SE peptide and CCN5, and were successfully introduced into both SMCs and ECs.

Meanwhile, NHCF-v was treated with TGF beta (TGFb) for 48 hours. Thereafter, the medium was treated with each experimental group construct for 48 hours, and the protein expression of a-SMA, p21, and GAPDH was confirmed. As a result, as shown in Figure 7, it was confirmed that a-SMA and p21, which were increased by TGFb, were reduced by the SE peptide and CCN5.

Furthermore, the mRNA expression of a-SMA, vimentin, and fibronectin in NHCF-v was confirmed using qRT-PCR. As a result, as shown in Figures 8 to 10, similar to the results of Western blot, it was confirmed that a-SMA, vimentin, and fibronectin, which are increased by TGFb, were decreased by the SE peptide and CCN5.

### Example 4.4. Confirmation of expression and extracellular secretion patterns of SE peptide and CCN5 according to structural changes in the gene construct

The expression and extracellular secretion patterns of the SE peptide and CCN5 were confirmed based on structural switching of the SE peptide and CCN5 and the position of the cell-penetrating peptide (CPP) of the SE peptide.

Specifically, AD293 cells were transfected with 3 µg of each of the following DNA plasmid constructs: i) CCN5-only expression, ii) N' (N-terminal)-CPP-SE and P2A-CCN5 multiple expression, iii) N'-CCN5-P2A and CPP-SE multiple expression, and iv) SE-CPP-C' (C-terminal) and P2A-CCN5 multiple expression. 24 hours after transfection, the expression of the SE peptide and the CCN5 protein in intracellular protein lysates and media was confirmed by Western blotting.

At this time, the structures of the constructs i), ii), iii), and iv) were CCN5-only, SPTATm3-SESE-P2A-CCN5, CCN5-P2A-SP-TATm3-SESE, and SP-SESE-TATm3-P2A-CCN5, respectively, in that order.

As a result, when the construct iii) CCN5-P2A-SP-TATm3-SESE was compared with the construct ii) SP-TATm3-SESE-P2A-CCN5 (BTG011), it was confirmed that the expression of the SE peptide and CCN5 within the cell was significantly reduced due to the structural switching of the SE peptide and CCN5 (Figure 11b). In addition, it was confirmed that the amounts of the SE peptide and CCN5 secreted into the medium were also significantly reduced (Figure 11c).

Furthermore, when the construct iv) SP-SESE-TATm3-P2A-CCN5 was compared with the construct ii) SP-TATm3-SESE-P2A-CCN5 (BTG011), it was confirmed that the expression of the SE peptide and CCN5 within the cell was slightly increased (Figure 11b), whereas the amounts of the SE peptide and CCN5 secreted into the medium were not increased (Figure 11c).

Meanwhile, it was confirmed that in the construct iv) SP-SESE-TATm3-P2A-CCN5, the expression of the SE peptide within the cell was increased (Figure 11b), whereas the secretion of the SE peptide into the medium was further reduced (Figure 11c). It was confirmed that the structural location of CPP affects the expression and secretion of the SE peptide and CCN5.

### Example 5. Confirmation of heart failure therapeutic effect in AngII heart failure mouse model

### Example 5.1. Production of AngII heart failure mouse animal model

In order to produce an angiotensin II (AngII) heart failure mouse animal model, C57BL/6 mice were injected with angiotensin II (AngII) at a dose of 3 mg/kg/day via an osmotic pump for 2 weeks.

### Example 5.2. Confirmation of heart failure therapeutic effect of gene construct injection

Six weeks after the production of the mice of Example 5.1. above, three types of AAV viruses, BTG001, BTG010, and BTG011, were injected at a dose of 1×10¹¹ vg, 5×10¹⁰ vg, 1×10¹⁰ vg, or 5×10⁹ vg, respectively. After six weeks, the cardiac contractility (fractional shortening) was confirmed through echocardiography.

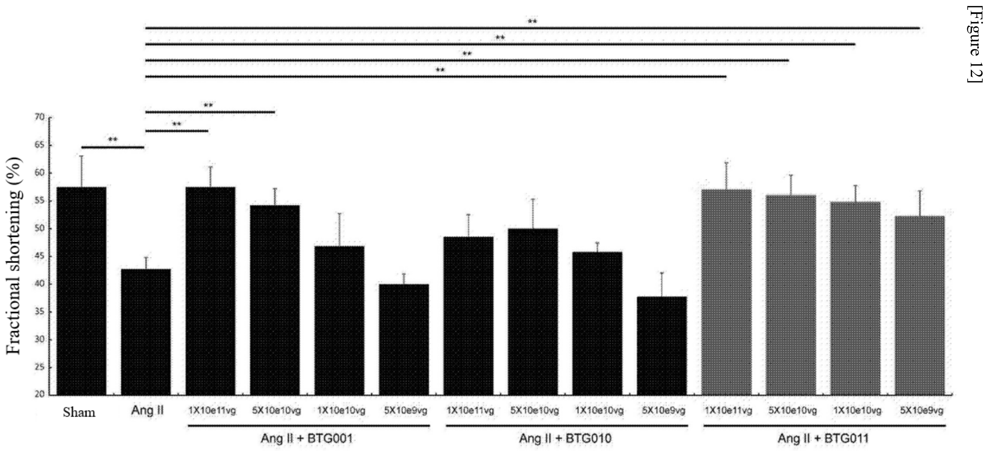

As a result, as shown in Figure 12, it was confirmed that the cardiac contractility was increased compared to the control group (AngII) in all mice injected with three types of AAV viruses, BTG001, BTG010, and BTG011. In particular, it was confirmed that in the mice injected with BTG011, the cardiac contractility was significantly increased at all doses, and the cardiac contractility was restored to the level of normal mice. These results suggest that the gene construct that simultaneously expresses the SPTATm3-SESE peptide and the CCN5 protein can effectively treat heart disease.

Furthermore, six weeks after gene construct injection, the degree of cardiac fibrosis was assessed using Trichrome staining, and the extent of fibrosis within the total tissue area was quantified using ImageScope.

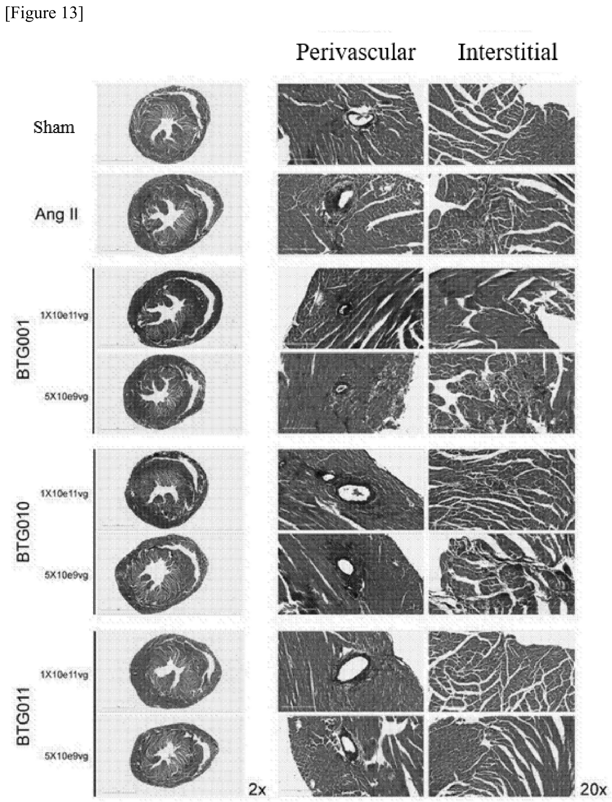

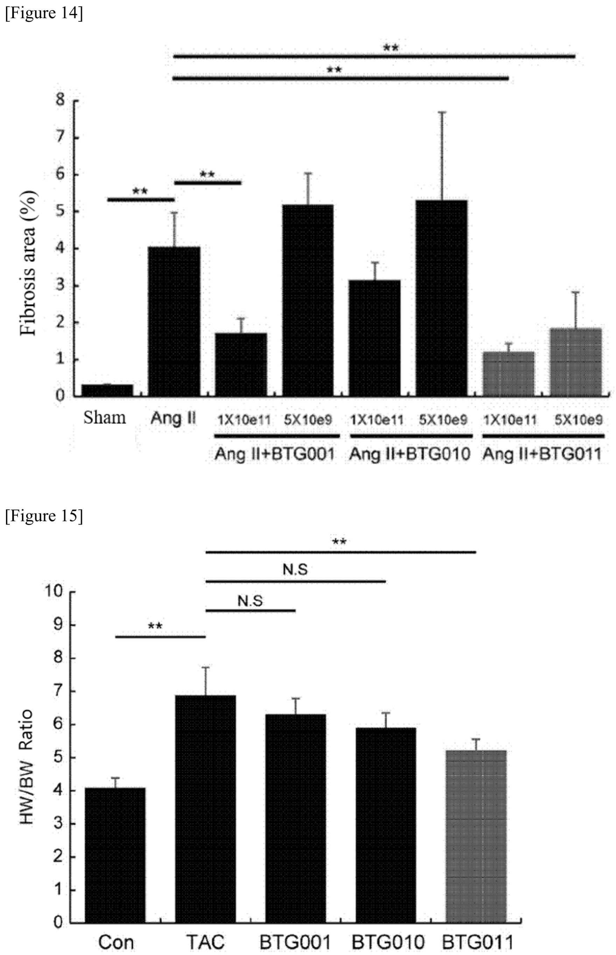

As a result, as shown in Figures 13 and 14, it was confirmed that the degree of cardiac fibrosis was reduced in all mice injected with three types of AAV viruses, BTG001, BTG010, and BTG011. In particular, it was confirmed that the fibrosis therapeutic effect was most pronounced in the mice injected with BTG011. These results suggest that the gene construct that simultaneously expresses the SPTATm3-SESE peptide and the CCN5 protein can effectively treat heart disease.

### Example 6. Confirmation of heart failure therapeutic effect in AngII heart failure mouse model

### Example 6.1. Production of transverse aortic constriction-induced heart failure mouse animal model

In order to produce an animal model of pressure overload (heart failure) due to transverse aortic constriction (TAC), 8 to 10-week-old C57BL/6 male mice (body weight of 25 to 30 g) were used in the study.

The mice were anesthetized by intraperitoneal injection with a solution containing 95 mg/kg ketamine and 5 mg/kg xylazine. The mice were ventilated using an oxygen respirator at a tidal volume of 0.2 and a respiratory rate of 11 breaths per minute (Harvard Apparatus). A 2-3 mm longitudinal incision was made in the manubrium to visualize the aortic arch. A 27-gauge needle was placed between the innominate artery and the left common carotid artery, and the transverse aortic arch was ligated. The needle was immediately removed, and the incision was covered to finish the procedure.

### Example 6.2. Confirmation of heart failure therapeutic effect of gene construct injection

Six weeks after the production of the mice of Example 6.1. above, three types of AAV viruses, BTG001, BTG010, and BTG011, were injected at a dose of 1×10¹⁰ vg, respectively. After six weeks, the heart weight and body weight of the mice were measured, and the cardiac contractility (fractional shortening) was confirmed through echocardiography.

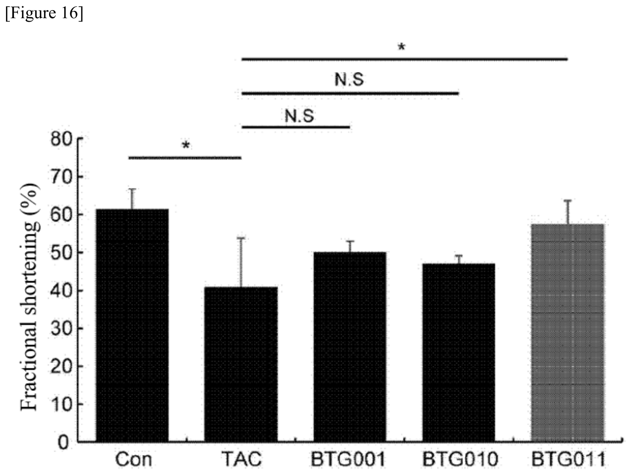

As a result, as shown in Figures 15 and 16, it was confirmed that in all mice injected with three types of AAV viruses (BTG001, BTG010, and BTG011), the heart weight/body weight ratio was lowered and the cardiac contractility was increased compared to the control group (TAC). In particular, in the mice injected with BTG011, a significant decrease in the heart weight (HW)/body weight (BW) ratio and a significant increase in the cardiac contractility were confirmed. These results suggest that the gene construct that simultaneously expresses the SPTATm3-SESE peptide and the CCN5 protein can effectively treat heart disease.

Furthermore, 6 weeks after gene construct injection, the degree of cardiac fibrosis was confirmed through trichrome staining, and the degree of fibrosis area among the total tissue area was quantified using Imagescope.

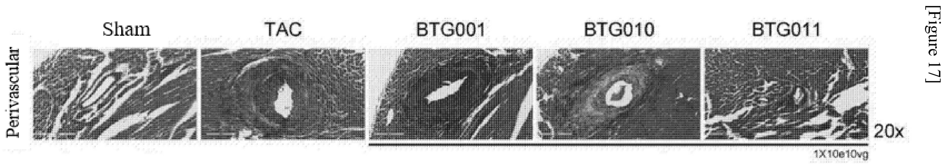

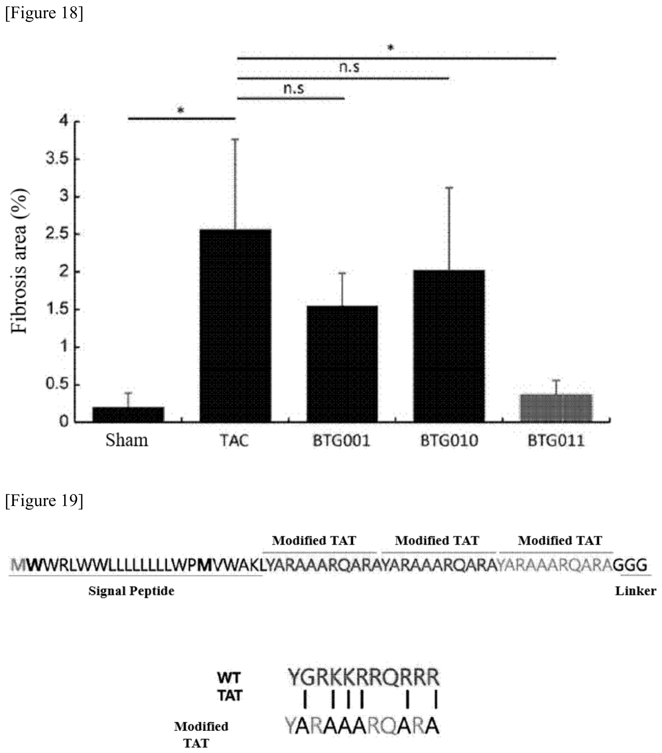

As a result, as shown in Figures 17 and 18, it was confirmed that in the mice injected with three types of AAV viruses (BTG001, BTG010, and BTG011), the degree of cardiac fibrosis was reduced compared to the control group (TAC). In particular, it was confirmed that the degree of cardiac fibrosis was most significantly reduced in the mice injected with BTG011. These results suggest that the gene construct that simultaneously expresses the SPTATm3-SESE peptide and the CCN5 protein can effectively treat heart disease.

### Sequence Listing

The sequences according to one embodiment of the present invention are listed in Table 4 below.

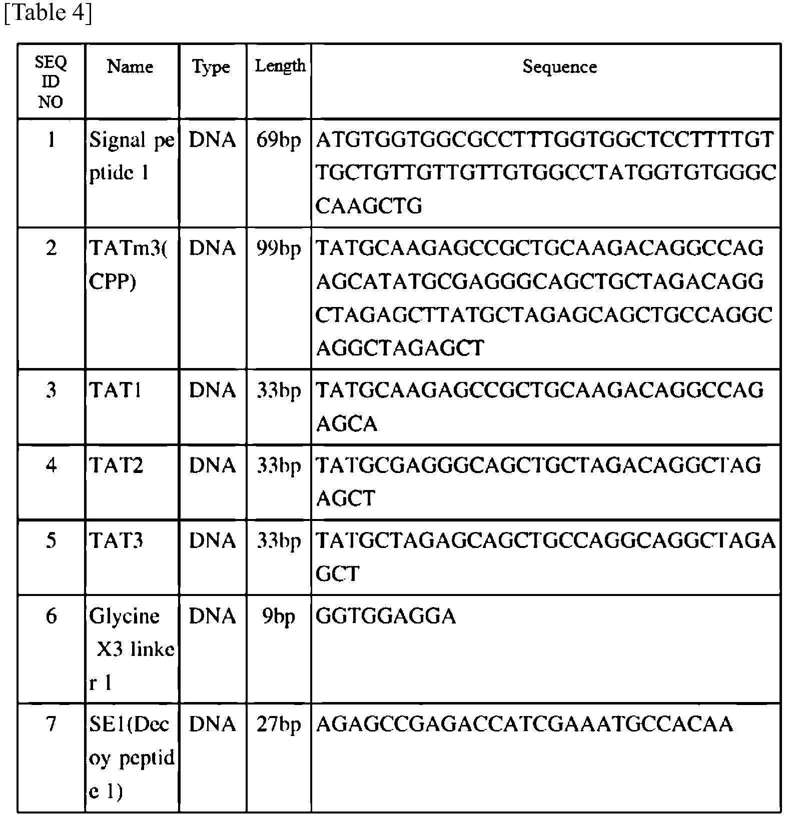

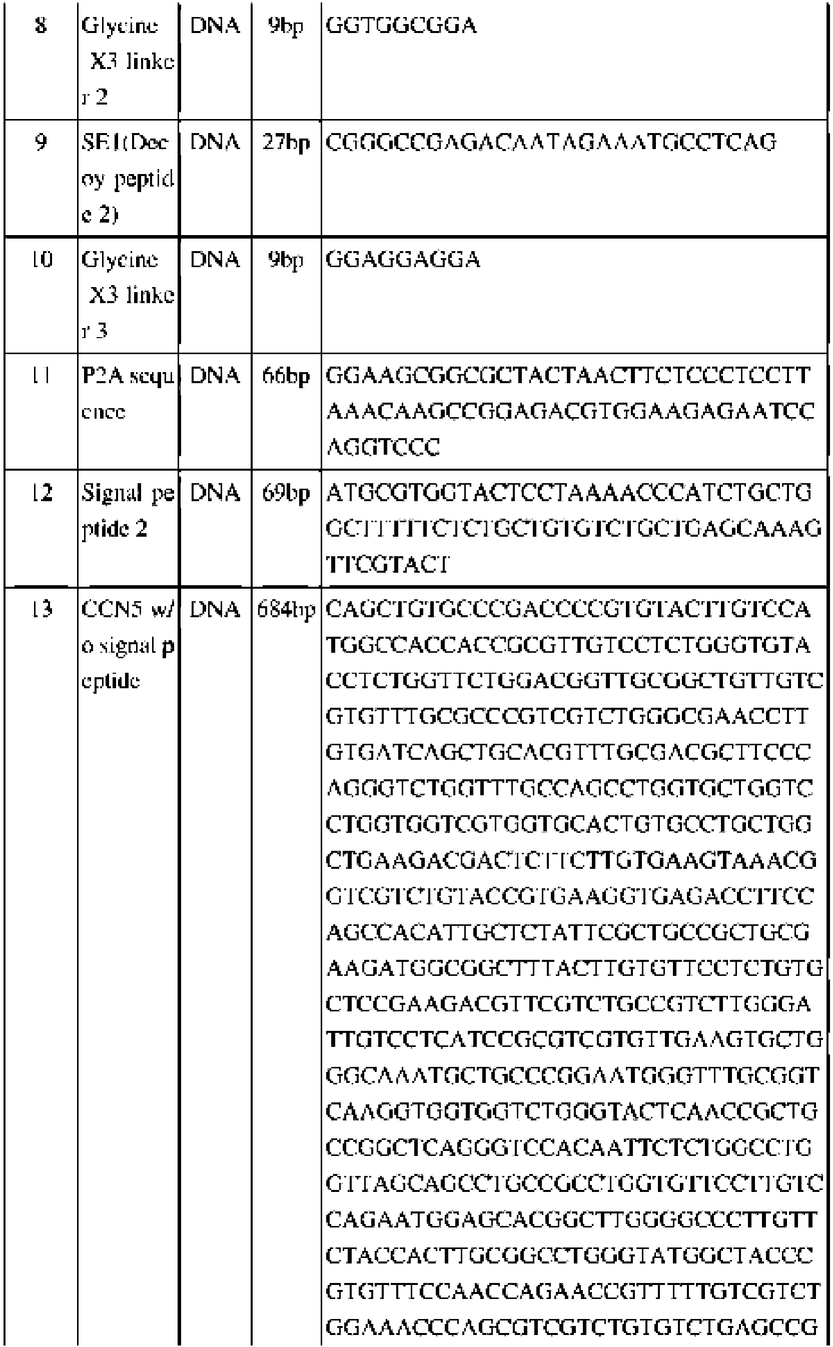

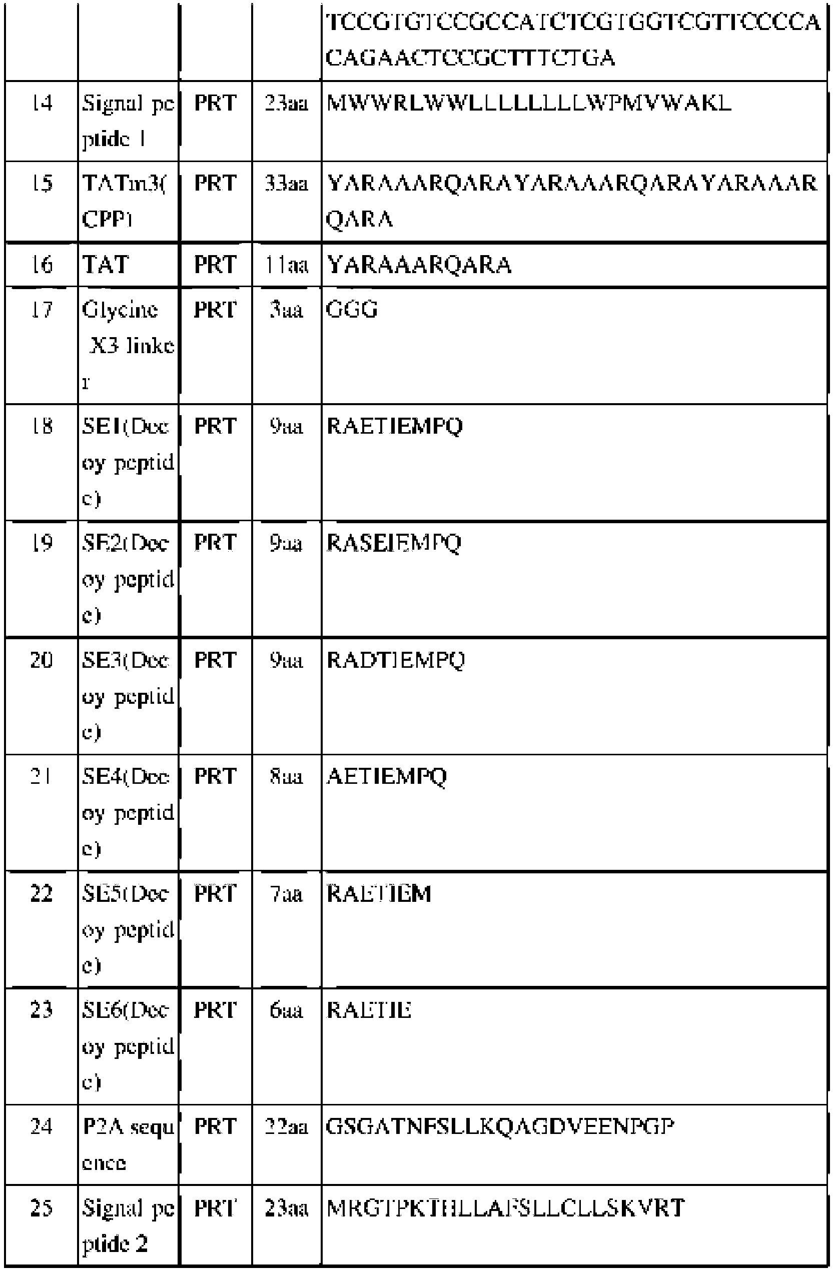

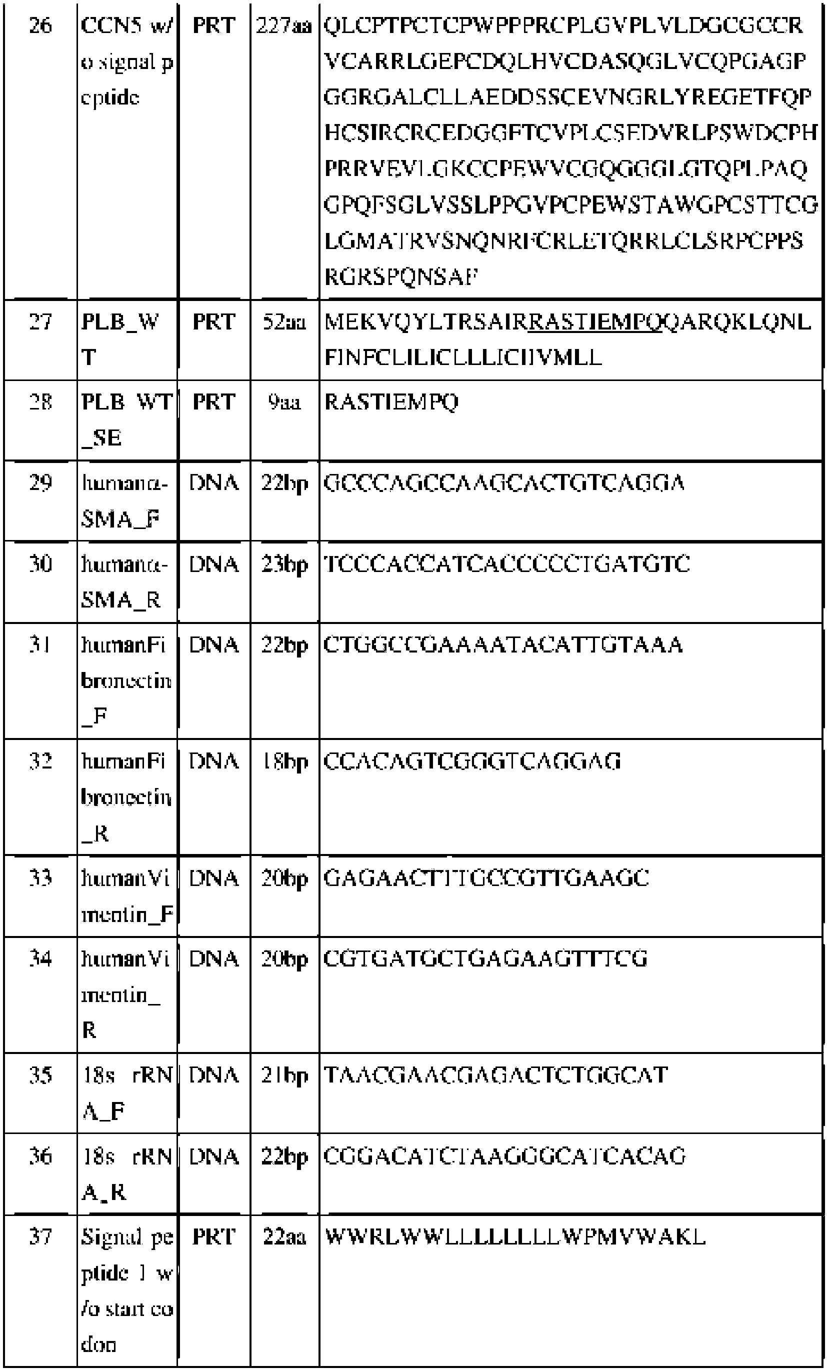

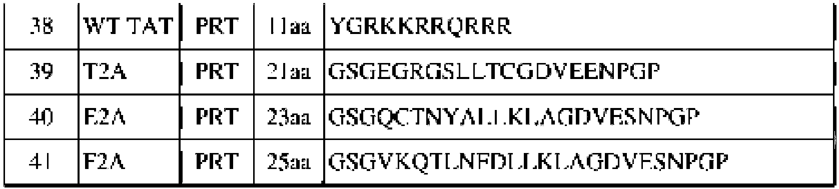

## Claims

1. A gene construct comprising a nucleotide encoding a decoy peptide that inhibits protein phosphatase 1 (PP1)-mediated dephosphorylation of phospholamban (PLB); and a nucleotide encoding a CCN5 protein or a fragment thereof.

2. The gene construct according to claim 1, wherein the decoy peptide consists of a peptide sequence represented by the following Structural formula (I):
X1-Ala-X2-X3-Ile-Glu-X4 (I)
wherein X1 is 0 to 50 amino acid residues,
X2 is Ser, Glu, or Asp,
X3 is Thr or Glu,
X4 is 0 to 50 amino acid residues, and
when X3 is Thr, X2 is not Ser.

3. The gene construct according to claim 2, wherein X1 is 0 to 13 amino acid residues.

4. The gene construct according to claim 2, wherein X4 is 0 to 33 amino acid residues.

5. The gene construct according to claim 3, wherein X1 is 0 to 1 amino acid residue.

6. The gene construct according to claim 4, wherein X4 is 0 to 3 amino acid residues.

7. The gene construct according to claim 5, wherein X1 is Arg.

8. The gene construct according to claim 6, wherein X4 is Met, Met-Pro, or Met-Pro-Gln.

9. The gene construct according to claim 2, wherein the decoy peptide comprises any one amino acid sequence selected from the group consisting of SEQ ID NOs: 18 to 23.

10. The gene construct according to claim 1, wherein the nucleotide encoding a decoy peptide and the nucleotide encoding a CCN5 protein or a fragment thereof are linked by a self-cleavage sequence.

11. The gene construct according to claim 10, wherein the self-cleavage sequence is a nucleotide sequence encoding a 2A peptide derived from porcine teschovirus-1 (PTV1), Thosea asigna virus (TaV), equine rhinitis A virus (ERAV), or foot and mouth disease virus (FMDV).

12. The gene construct according to claim 11, wherein the self-cleavage sequence has the amino acid sequence of SEQ ID NO: 24, 39, 40, or 41.

13. The gene construct according to claim 1, wherein a nucleotide encoding a cell-penetrating peptide is additionally linked to the gene construct.

14. The gene construct according to claim 13, wherein the cell-penetrating peptide is selected from the group consisting of a Tat-derived peptide, an arginine-rich peptide, a transportan, and an amphipathic peptide carrier.

15. The gene construct according to claim 1, wherein the gene construct further comprises the nucleotide encoding a decoy peptide.

16. The gene construct according to claim 1, wherein the CCN5 protein has the amino acid sequence of SEQ ID NO: 26.

17. The gene construct according to claim 1, wherein the gene construct consists of Structural formula (II) below:
[ SP1 - X - Y - ((L1)p - Y)n- (L2)q - Z ]m - SP2 - T (II)
wherein, in Structural formula (II),
X is the nucleotide encoding a cell-penetrating peptide,
Y is the nucleotide encoding a decoy peptide,
SP1 and SP2 are nucleotides encoding a signal sequence,
L1 and L2 are peptide linkers,
Z is a nucleotide encoding the cleavage sequence,
T is the nucleotide encoding a CCN5 protein or a fragment thereof,
n is any one integer from 0 to 3,
m is any one integer from 1 to 3, and
p and q are each 0 or 1.

18. The gene construct according to claim 1, wherein the gene construct comprises a signal sequence.

19. A recombinant vector comprising the gene construct according to any one of claims 1 to 18.

20. A recombinant virus comprising the gene construct according to any one of claims 1 to 18.

21. The recombinant virus according to claim 20, wherein the virus is any one selected from the group consisting of adenovirus, adeno-associated virus (AAV), retrovirus, lentivirus, herpes simplex virus, anellovirus, and vaccinia virus.

22. A pharmaceutical composition comprising the gene construct according to claim 1, the recombinant vector according to claim 19, or the recombinant virus according to claim 20 as an active ingredient.

23. The pharmaceutical composition according to claim 22, wherein the pharmaceutical composition is for the prevention or treatment of a heart disease.

24. The pharmaceutical composition according to claim 23, wherein the heart disease is selected from the group consisting of heart failure, ischemia, arrhythmia, myocardial infarction, congestive heart failure, transplant rejection, abnormal cardiac contraction, and abnormal Ca²⁺ metabolism.

25. A method for preventing or treating a heart disease, comprising administering the pharmaceutical composition according to claim 22 to a subject.

26. A use of a pharmaceutical composition comprising the gene construct according to claim 1, the recombinant vector according to claim 19, or the recombinant virus according to claim 20 as an active ingredient for the prevention or treatment of a heart disease.

27. A use of a pharmaceutical composition comprising the gene construct according to claim 1, the recombinant vector according to claim 19, or the recombinant virus according to claim 20 as an active ingredient for the manufacture of a medicament for preventing or treating a heart disease.
